# EUROPEAN PATENT APPLICATION

(11) **EP 1 783 223 A1**
(43) Date of publication of application: **09.05.2007**
(21) Application number: 06126806.6
(22) Date of filing: 05.10.1999
(51) Int. Cl.: C12N 15/31, C12N 15/80, C12N 1/15, C07K 14/38, C12R 1/685

(54) **Fungal transcriptional activator PRTT from Aspergillus niger, useful in methods for producing polypeptides**

(30) Priority: 05.10.1998 DK 125898
(62) Divisional of application: 99945966.2
(71) Applicant: Novozymes A/S, 2880 Bagsvaerd (DK)
(72) Inventor: Hjort, Carsten, 3500 Vaerloese (DK); Van den Hondel, Cees, A., M., J., J., 2804 PZ Gouda (NL); Punt, Peter, J., 3994 XT Houten (NL); Schuren, Frank, H., J., 3906 ZK Veenendaal (NL)

(57) **Abstract**

The present invention relates to isolated nucleic acid sequences encoding polypeptides having transcriptional activation activity and to the polypeptides. The invention also relates to nucleic acid constructs, vectors and host cells comprising the nucleic acid sequences. The invention further relates to host cells useful for the production of polypeptides in which the production or function of the transcriptional activator has been altered, as well as to methods for producing the polypeptides.

SEQUENCE LISTING

<110> Novozymes A/S
      Krogshøjvej 36
      2880 Bagsværd
      Denmark

<120>  Fungal transcriptional activator prtT from Aspergillus niger, Useful in
methods for producing polypeptides

<130>  5555.215-EP

<160>  33

<170>  PatentIn version 3.0

<210>  1
<211>  4098
<212>  DNA
<213>  Aspergillus niger

<220>
<221>  exon
<222>  (977)..(1204)

<220>
<221>  exon
<222>  (1317)..(1718)

<220>
<221>  exon
<222>  (1777)..(2202)

<220>
<221>  exon
<222>  (2253)..(3116)

<220>
<221>  exon
<222>  (3173)..(3248)

<400>  1
TTGGTGCTGG AAAGCCCATT TAAGGGATCT TATAAGGTAA TTGCCAATGT TCAGTCGCCT      60

ATGGTCTTTG TCGAGAGAAA CTCTTTCTCG TTAAGATCTA CATGATCGCT TTTGATTTTC     120

TCTGGGTTCA CGCGGTACTT TCTCCCCGTC AATCCCCAAC CGCTGTTGTG CCTGACCATC     180

AATGTGGAAC GGATAAGGGG ACAAGAGAAA TTGAAGGAGC GATCATAAAA AGCTAATTTT     240

GGTTTATTAT TTTTTTTTCT TATAAAACTC AAAAAAGAAA ACGAAAACGA AAAAGGAAAA     300

AAGAAAAGGT AAAATGGAAA AAGAAAGGCG GTCATCACTT CCAATAACCA TCAGCCAAAG     360

ATACAGACGA GTTACTGACC TTCTTATCCT GGACTTCCGC CCGATCCATA TCTTCATGAT     420

AAGCAGGGAA CCGAACAAAT CAACGCCAAC TTCAGCGGCA GTTCCTCACT AATTTCCCAC     480

TTCCCACCGG CGTCATTTTG GTCCCAACCC CCTCCCTGGA AGCAGCGGGA TTTAGTTACG     540

ATCCGGTTTA CATCGGAGAC TCGGAAAATA CCATAGCGCA TGCCAATCAA AACCCCTCCC     600

AGGGTGACTG GCCAGTATCA CGACCCATTG TTTCTATCTT TCTAGAAGAC CTGCAGGGAC 660

ATGGATTGGC TGGCCGCCGT GCTGCCGTCC ATTAGCGTCT ACCCCAGGTC AAGAACGGAC 720

TGGACGGACC CATAACCAAT CTAACCAAAG CCAATTTCGT CAATTCCCAG CTGGCGAGCA 780

CAATCCCATT CCCAGGGTTG GCCGCCAACT GTTAAAAGGC ACTATGTGTC TCTCCACCTG 840

CCCGCCCCCC TCGATGGCCT GCGCGTAATA ACTATTCTAC TGCTTTTTGC CTCTTACTTG 900

CCTCATTATT AGTATTTTAC TCTACTCTCC AGATTGCCTG CCAGCAATTG GTCCAAAGTG 960

GACTTTGTTT GATGACATGA CTCGAACCGT GGACGAGATC AAATACGAAA CGCCTTCTTC 1020

ATGGGAGCAC AAGAGCTTGG ACGTTGCCGA GGATGGCAGG CGACTAGCTC CCCATTCCGA 1080

CACTGCTCGT CCGAAAGGCC GCATACGACG ATCGATGACT GCCTGTCACA CATGTCGGAA 1140

GCTTAAAACT AGATGTGATC TAGATCCGCG CGGTCATGCG TGCCGTCGCT GTCTATCTCT 1200

AAGGTCAGAG GCACTACCTA CCTGCCAGTT GAAGCTTTGT CCTTCTGAAC GCGACATGAT 1260

ACTAGTCGTG GAATATAACT GTCCCAACTT TGCTGACAGT CCACAATATC TTTAGAATCG 1320

ATTGTAAGCT GCCTGAAACG ACCGACCGCT TCCAAGACAG TGCTGCGATG TGGCCAGACG 1380

CCACCTCGGC AATTCCCTCC ATCGAGGAGC GCCTCACCTC CCTAGAAAGA TGCATGAGGG 1440

AGATGACGGG CATGATGCGA CAGATGCTAG ATCACTCCCC AGGTTTCGCA AATGCCTCGG 1500

TTCCGCATTT GACCAAAAGC ATCATCACGG ATGAAACCGC CTCGATGGAG GGAAGCCCGT 1560

CGTCCCCCTT CCTGCCTAAG CCCGTTCGCC TCATTCAGGA CCTCCAGTCC GACTTCTTCG 1620

GAGAAGCAGA GACTTCCCCC GTTGACTCCC CTCTCTCCAG CGATGGTAAC GCCAAGGGCG 1680

CTATCGACTC TAAGCTATCC CTCAAATTGT TGCAAACGTA TGGGTATACC TGATTGACAA 1740

TTACCAAAAA GCTGCTAATC CTTGGCGCAA ATCAGGTTTG TCGATCACTT TGGCGCTTGC 1800

GTTTCCATTT ACAATCTCTC CGACATCCAC AACGACATGA AAGCCCCCGA CTCTTTACTG 1860

TATAATACTG CATGCCTTCT AGCTTCACGC TATGTACCGG GGATACCGAC ATCTACCGTG 1920

CATGCTATAT ACCTTCAAGT GCGACATGCA GTAGTCAATA TTTTGTGGGA AAAACCACCC 1980

CTGAAGTATG AGACCCTCCA AGCACTTGCA CTTCTCTGTC TCTGGCCAGC AACCGCCCAG 2040

AAAGAGCCAC CCATGGACAG CTGGCTGCTG AGTGGTATCT CAATTAACCA TGCAATTATC 2100

GCGCTCGATT TCCTAAACTA TGCGCCCTCG GAAGTCATGG TGGACAATGA AACGGCTGCG 2160

CAGCTGCGGC TATGGAATAC ATATTGCTTG ACACAGCTAC AGTGGGTTTC ATCTAAGATC 2220

TCCCGTCCAG AAGATAGCTA ACAAGCTTTA GTTTTGCGGT CGGGAATGCG CGTCCTTTCC 2280

ATATCCAGCA AAGATACCTT GACCACTGCC CACGGATACT GGAGCACCCA GCAGCAACTC 2340

TGGAGGACGC AAGGGTTGTA GCAGAAATAC AGTTGTATTT GATGACATTG CGGCTCCAGA 2400

GCAATAGCAG TCGAATGCGG TTGGCGGACC TTGACTATGA GGAAATAGAG CGATGGAAGA 2460

GGGAGTGGGC TCACCTTTTC TGTAAGAAGC CTGTTCTTGT TTCCCGGGGA CTACCACTGA 2520

CGAGAGCAAC AGCTGGGGAA AGTTCCACAT TGGAGCTGAG CCTTTGGTTC TGCCAGACAC 2580

TCCTTCACCG CACAGCAATG AGGCTTCAGC CCAGATCCGA CAGGCTCGCA TCTGAGGTTC 2640

TGCAAACCTC ACGTCTGATA ATATCGCGGT TCCTCCAGAT CCGGTACTCT ACCGCATTAA 2700

GCCTTGTCGA CCAAGTCTAT TTCATTGTCG GCTACGCTGC ACTGAATCTG TGCGATTTCA 2760

ATCTTATGGA CCCGCTTATC GAGCAAGTGC AGATGTTCCT GCTGCATCTC TCCCCGAACG 2820

AAGACCACAT CGCCTACCGG TTTTCGTGCA TGGTCGCCGA GTTCAAGCGG CGATGTGGCA 2880

GTGCGGAATG CAATGACCCA TCATCCACTG TCAAGGGGTC TCCGTTATCA TCCTACGGCG 2940

ACAGTCGTAA GATGAGCATG GGGCAAGCAC CGTTCATGCC ACCGCTCATG GATGGCATGA 3000

TCGAGGGGTA CGGCTTCGAG CAACTGATGC CAGAAGTCAT GCCGAGTTCC TTTCCGGATG 3060

GGATACTCAA CGGAATGCCT GTGACTGGGC TAGCAGCGTA TCGGTCAGCG ACGCTGTAAG 3120

TAATCGAGAT CGGGTTGGAA AGGACATGAG TGGGGGTGGT GGTGGTAGTA GCAGTAACAC 3180

CAGGGATGAT AACCTGCAGC GGTGGTTTAG TTCCTGCCCA TGGGCTGAAC TAAAACCCCG 3240

AACCTAGCAT GATGACGTGC AACGAAAGGA TCATAACCAA GGCCAAGTAA ATACTAAAAT 3300

AAAATAATAT AATTCCACAC GATCCACTAC CACCACCACC ACCGGATCCA TCAGGTTGCC 3360

TTCCTGCACA GGCCTATTTA GTTAGAGGGC CCGTGCCACG AAACATCACG TAATTGAGCG 3420

CTTTTGCTTC CTTGCAACTT AAACAACCCC ATAGACACTC TCACATTCAC ATGCCAAACT 3480

ACTAACTCCT ACTGACCACC AGCTGCAGGA AGCCAGCCAG CCACCATTTC CTAATCGGAT 3540

ATATCTCCGA AACGTACGCT TTCCTCCTTT GTTCGGACCG TTCCGTGCCT CCGCGGAGAG 3600

TTGAACGAGT CAGAACACAT TCTTTTCGTT TCTATCGTTT CTTTTCCAAG GCAGCAGAGA 3660

GACGAACAAG TCAGTGCTTG CTAACTAACT TACCCCTCAG CATTTTAGTA AACTACTATT 3720

TAGGAAAGAG TAATCATTCA TCGAAGACAA GATGTTTATT TCTCCGATCG ACCAAACAAA 3780

AACGTTCAGG TAGACTAAGT AGTAGTAGTA GTATGTCTTT GACCCCTTTA CTCCACTATC 3840

CGTTGACTGC ACATAGTAGT AAGTAACTAT CTAACCAGTT GCCGAGGAGA GGAAAGTGAG 3900

TGGGTGGGAG CCGGAGGATG CCGCCGAGAA TTATTAAGTC GATCATTGCT AGTTAGTTAT 3960

CTTTTCATGA TGAGGAGAGG AAGGAGAGGG GGGACGGGAT TAGAGAAATA AACTTTTCTC 4020

TCCAATTAAT TATCTGGATT AATTAAAACT TGGAGAGGAG GGTAGGGGAG TTGGGTATTG 4080

GTATGTTGCT GTGAATGT 4098

<210> 2
<211> 666

<212>  PRT
<213>  Aspergillus niger

<400>  2

Met Thr Arg Thr Val Asp Glu Ile Lys Tyr Glu Thr Pro Ser Ser Trp
1               5                   10                  15

Glu His Lys Ser Leu Asp Val Ala Glu Asp Gly Arg Arg Leu Ala Pro
            20                  25                  30

His Ser Asp Thr Ala Arg Pro Lys Gly Arg Ile Arg Arg Ser Met Thr
        35                  40                  45

Ala Cys His Thr Cys Arg Lys Leu Lys Thr Arg Cys Asp Leu Asp Pro
    50                  55                  60

Arg Gly His Ala Cys Arg Arg Cys Leu Ser Leu Arg Ile Asp Cys Lys
65                  70                  75                  80

Leu Pro Glu Thr Thr Asp Arg Phe Gln Asp Ser Ala Ala Met Trp Pro
                85                  90                  95

Asp Ala Thr Ser Ala Ile Pro Ser Ile Glu Glu Arg Leu Thr Ser Leu
            100                 105                 110

Glu Arg Cys Met Arg Glu Met Thr Gly Met Met Arg Gln Met Leu Asp
        115                 120                 125

His Ser Pro Gly Phe Ala Asn Ala Ser Val Pro His Leu Thr Lys Ser
    130                 135                 140

Ile Ile Thr Asp Glu Thr Ala Ser Met Glu Gly Ser Pro Ser Ser Pro
145                 150                 155                 160

Phe Leu Pro Lys Pro Val Arg Leu Ile Gln Asp Leu Gln Ser Asp Phe
                165                 170                 175

Phe Gly Glu Ala Glu Thr Ser Pro Val Asp Ser Pro Leu Ser Ser Asp
            180                 185                 190

Gly Asn Ala Lys Gly Ala Ile Asp Ser Lys Leu Ser Leu Lys Leu Leu
        195                 200                 205

Gln Thr Phe Val Asp His Phe Gly Ala Cys Val Ser Ile Tyr Asn Leu
    210                 215                 220

Ser Asp Ile His Asn Asp Met Lys Ala Pro Asp Ser Leu Leu Tyr Asn
225                 230                 235                 240

Thr Ala Cys Leu Leu Ala Ser Arg Tyr Val Pro Gly Ile Pro Thr Ser
                245                 250                 255

Thr Val His Ala Ile Tyr Leu Gln Val Arg His Ala Val Val Asn Ile
            260                 265                 270

Leu Trp Glu Lys Pro Pro Leu Lys Tyr Glu Thr Leu Gln Ala Leu Ala
        275                 280                 285

Leu Leu Cys Leu Trp Pro Ala Thr Ala Gln Lys Glu Pro Pro Met Asp
    290                 295                 300

Ser Trp Leu Leu Ser Gly Ile Ser Ile Asn His Ala Ile Ile Ala Leu
305                 310                 315                 320

Asp Phe Leu Asn Tyr Ala Pro Ser Glu Val Met Val Asp Asn Glu Thr
                325                 330                 335

Ala Ala Gln Leu Arg Leu Trp Asn Thr Tyr Cys Leu Thr Gln Leu His
            340                 345                 350

Phe Ala Val Gly Asn Ala Arg Pro Phe His Ile Gln Gln Arg Tyr Leu
        355                 360                 365

Asp His Cys Pro Arg Ile Leu Glu His Pro Ala Ala Thr Leu Glu Asp
    370                 375                 380

Ala Arg Val Val Ala Glu Ile Gln Leu Tyr Leu Met Thr Leu Arg Leu
385                 390                 395                 400

Gln Ser Asn Ser Ser Arg Met Arg Leu Ala Asp Leu Asp Tyr Glu Glu
                405                 410                 415

Ile Glu Arg Trp Lys Arg Glu Trp Ala His Leu Phe Cys Lys Lys Pro
            420                 425                 430

Val Leu Val Ser Arg Gly Leu Pro Leu Thr Arg Ala Thr Ala Gly Glu
        435                 440                 445

Ser Ser Thr Leu Glu Leu Ser Leu Trp Phe Cys Gln Thr Leu Leu His
    450                 455                 460

Arg Thr Ala Met Arg Leu Gln Pro Arg Ser Asp Arg Leu Ala Ser Glu
465                 470                 475                 480

Val Leu Gln Thr Ser Arg Leu Ile Ile Ser Arg Phe Leu Gln Ile Arg
                485                 490                 495

Tyr Ser Thr Ala Leu Ser Leu Val Asp Gln Val Tyr Phe Ile Val Gly
            500                 505                 510

Tyr Ala Ala Leu Asn Leu Cys Asp Phe Asn Leu Met Asp Pro Leu Ile
        515                 520                 525

Glu Gln Val Gln Met Phe Leu Leu His Leu Ser Pro Asn Glu Asp His
    530                 535                 540

Ile Ala Tyr Arg Phe Ser Cys Met Val Ala Glu Phe Lys Arg Arg Cys
545                 550                 555                 560

Gly Ser Ala Glu Cys Asn Asp Pro Ser Ser Thr Val Lys Gly Ser Pro
                565                 570                 575

Leu Ser Ser Tyr Gly Asp Ser Arg Lys Met Ser Met Gly Gln Ala Pro
            580                 585                 590

Phe Met Pro Pro Leu Met Asp Gly Met Ile Glu Gly Tyr Gly Phe Glu
        595                 600                 605

Gln Leu Met Pro Glu Val Met Pro Ser Ser Phe Pro Asp Gly Ile Leu
    610                 615                 620

Asn Gly Met Pro Val Thr Gly Leu Ala Ala Tyr Arg Ser Ala Thr Leu
625                 630                 635                 640

Ser Ser Asn Thr Arg Asp Asp Asn Leu Gln Arg Trp Phe Ser Ser Cys
                645                 650                 655

Pro Trp Ala Glu Leu Lys Pro Arg Thr Pro
            660                 665

<210>  3
<211>  35
<212>  PRT
<213>  Aspergillus niger

<400>  3

Met Thr Ala Cys His Thr Cys Arg Lys Leu Lys Thr Arg Cys Asp Leu
1               5                   10                  15

Asp Pro Arg Gly His Ala Cys Arg Arg Cys Leu Ser Leu Arg Ile Asp
            20                  25                  30

Cys Lys Leu
        35

<210>  4
<211>  2542
<212>  DNA
<213>  Artificial

<220>
<223>  Synthetic

<400>  4
gacatggatt ggctggccgc cgtgctgccg tccattagcg tctaccccag gtcaagaacg      60

gactggacgg acccataacc aatctaacca aagccaattt cgtcaattcc cagctggcga     120

gcacaatccc attcccaggg ttggccgcca actgttaaaa ggcactatgt gtctctccac     180

ctgcccgccc ccctcgatgg cctgcgcgta ataactattc tactgctttt tgcctcttac     240

ttgcctcatt attagtattt tactctactc tccagattgc ctgccagcaa ttggtccaaa     300

gtggactttg tttgatgaca tgactcgaac cgtggacgag atcaaatacg aaacgccttc     360

ttcatgggag cacaagagct tggacgttgc cgaggatggc aggcgactag ctccccattc     420

cgacactgct cgtccgaaag gccgcatacg acgatcgatg actgcctgtc acacatgtcg     480

gaagcttaaa actagatgtg atctagatcc gcgcggtcat gcgtgccgtc gctgtctatc     540

tctaaggtca gaggcactac ctacctgcca gttgaagctt tgtccttctg aacgcgacat     600

gatactagtc gtggaatata actgtcccaa ctttgctgac agtccacaat atctttagaa     660

tcgattgtaa gctgcctgaa acgaccgacc gcttccaaga cagtgctgcg atgtggccag     720

acgccacctc ggcaattccc tccatcgagg agcgcctcac ctccctagaa agatgcatga     780

gggagatgac gggcatgatg cgacagatgc tagatcactc cccaggtttc gcaaatgcct     840

cggttccgca tttgaccaaa agcatcatca cggatgaaac cgcctcgatg gagggaagcc 900

cgtcgtcccc cttcctgcct aagcccgttc gcctcattca ggacctccag tccgacttct 960

tcggagaagc agagacttcc cccgttgact cccctctctc cagcgatggt aacgccaagg 1020

gcgctatcga ctctaagcta tccctcaaat tgttgcaaac gtatgggtat acctgattga 1080

caattaccaa aaagctgcta atccttggcg caaatcaggt ttgtcgatca ctttggcgct 1140

tgcgtttcca tttacaatct ctccgacatc cacaacgaca tgaaagcccc cgactcttta 1200

ctgtataata ctgcatgcct tctagcttca cgctatgtac cggggatacc gacatctacc 1260

gtgcatgcta tataccttca agtgcgacat gcagtagtca atattttgtg ggaaaaacca 1320

cccctgaagt atgagaccct ccaagcactt gcacttctct gtctctggcc agcaaccgcc 1380

cagaaagagc cacccatgga cagctggctg ctgagtggta tctcaattaa ccatgcaatt 1440

atcgcgctcg atttcctaaa ctatgcgccc tcggaagtca tggtggacaa tgaaacggct 1500

gcgcagctgc ggctatggaa tacatattgc ttgacacagc tacagtgggt ttcatctaag 1560

atctcccgtc cagaagatag ctaacaagct ttagttttgc ggtcgggaat gcgcgtcctt 1620

tccatatcca gcaaagatac cttgaccact gcccacggat actggagcac ccagcagcaa 1680

ctctggagga cgcaagggtt gtagcagaaa tacagttgta tttgatgaca ttgcggctcc 1740

agagcaatag cagtcgaatg cggttggcgg accttgacta tgaggaaata gagcgatgga 1800

agagggagtg ggctcacctt ttctgtaaga agcctgttct tgtttcccgg ggactaccac 1860

tgacgagagc aacagctggg gaaagttcca cattggagct gagcctttgg ttctgccaga 1920

cactccttca ccgcacagca atgaggcttc agcccagatc cgacaggctc gcatctgagg 1980

ttctgcaaac ctcacgtctg ataatatcgc ggttcctcca gatccggtac tctaccgcat 2040

taagccttgt cgaccaagtc tatttcattg tcggctacgc tgcactgaat ctgtgcgatt 2100

tcaatcttat ggacccgctt atcgagcaag tgcagatgtt cctgctgcat ctctccccga 2160

acgaagacca catcgcctac cggttttcgt gcatggtcgc cgagttcaag cggcgatgtg 2220

gcagtgcgga atgcaatgac ccatcatcca ctgtcaaggg gtctccgtta tcatcctacg 2280

gcgacagtcg taagatgagc atggggcaag caccgttcat gccaccgctc atggatggca 2340

tgatcgaggg gtacggcttc gagcaactga tgccagaagt catgccgagt tcctttccgg 2400

atgggatact caacggaatg cctgtgactg ggctagcagc gtatcggtca gcgacgctgt 2460

aagtaatcga gatcgggttg gaaaggacat gagtgggggt ggtggtggta gtagcagtaa 2520

caccagggat gataacctgc ag 2542

<210> 5

<211>  665
<212>  PRT
<213>  Artificial

<220>
<223>  Synthetic

<400>  5

Met Thr Arg Thr Val Asp Glu Ile Lys Tyr Glu Thr Pro Ser Ser Trp
1               5                   10                  15

Glu His Lys Ser Leu Asp Val Ala Glu Asp Gly Arg Arg Leu Ala Pro
            20                  25                  30

His Ser Asp Thr Ala Arg Pro Lys Gly Arg Ile Arg Arg Ser Met Thr
        35                  40                  45

Ala Cys His Thr Cys Arg Lys Leu Lys Thr Arg Cys Asp Leu Asp Pro
    50                  55                  60

Arg Gly His Ala Cys Arg Arg Cys Leu Ser Leu Arg Ile Asp Cys Lys
65                  70                  75                  80

Leu Pro Glu Thr Thr Asp Arg Phe Gln Asp Ser Ala Ala Met Trp Pro
                85                  90                  95

Asp Ala Thr Ser Ala Ile Pro Ser Ile Glu Glu Arg Leu Thr Ser Pro
            100                 105                 110

Glu Arg Cys Met Arg Glu Met Thr Gly Met Met Arg Gln Met Leu Asp
        115                 120                 125

His Ser Pro Gly Phe Ala Asn Ala Ser Val Pro His Leu Thr Lys Ser
    130                 135                 140

Ile Ile Thr Asp Glu Thr Ala Ser Met Glu Gly Ser Pro Ser Ser Pro
145                 150                 155                 160

Phe Leu Pro Lys Pro Val Arg Leu Ile Gln Asp Leu Gln Ser Asp Phe
                165                 170                 175

Phe Gly Glu Ala Glu Thr Ser Pro Val Asp Ser Pro Leu Ser Ser Asp
            180                 185                 190

Gly Asn Ala Lys Gly Ala Ile Asp Ser Lys Leu Ser Leu Lys Leu Leu
        195                 200                 205

Gln Thr Phe Val Asp His Phe Gly Ala Cys Val Ser Ile Tyr Asn Leu
    210                 215                 220

Ser Asp Ile His Asn Asp Met Lys Ala Pro Asp Ser Leu Leu Tyr Asn
225                 230                 235                 240

Thr Ala Cys Leu Leu Ala Ser Arg Tyr Val Pro Gly Ile Pro Thr Ser
                245                 250                 255

Thr Val His Ala Ile Tyr Leu Gln Val Arg His Ala Val Val Asn Ile
            260                 265                 270

Leu Trp Glu Lys Pro Pro Leu Lys Tyr Glu Thr Leu Gln Ala Leu Ala

275                 280                 285

Leu Leu Cys Leu Trp Pro Ala Thr Ala Gln Lys Glu Pro Pro Met Asp
    290                 295                 300

Ser Trp Leu Leu Ser Gly Ile Ser Ile Asn His Ala Ile Ile Ala Leu
305                 310                 315                 320

Asp Phe Leu Asn Tyr Ala Pro Ser Glu Val Met Val Asp Asn Glu Thr
                325                 330                 335

Ala Ala Gln Leu Arg Leu Trp Asn Thr Tyr Cys Leu Thr Gln Leu His
            340                 345                 350

Phe Ala Val Gly Asn Ala Arg Pro Phe His Ile Gln Gln Arg Tyr Leu
        355                 360                 365

Asp His Cys Pro Arg Ile Leu Glu His Pro Ala Ala Thr Leu Glu Asp
    370                 375                 380

Ala Arg Val Val Ala Glu Ile Gln Leu Tyr Leu Met Thr Leu Arg Leu
385                 390                 395                 400

Gln Ser Asn Ser Ser Arg Met Arg Leu Ala Asp Leu Asp Tyr Glu Glu
                405                 410                 415

Ile Glu Arg Trp Lys Arg Glu Trp Ala His Leu Phe Cys Lys Lys Pro
            420                 425                 430

Val Leu Val Ser Arg Gly Leu Pro Leu Thr Arg Ala Thr Ala Gly Glu
        435                 440                 445

Ser Ser Thr Leu Glu Leu Ser Leu Trp Phe Cys Gln Thr Leu Leu His
    450                 455                 460

Arg Thr Ala Met Arg Leu Gln Pro Arg Ser Asp Arg Leu Ala Ser Glu
465                 470                 475                 480

Val Leu Gln Thr Ser Arg Leu Ile Ile Ser Arg Phe Leu Gln Ile Arg
                485                 490                 495

Tyr Ser Thr Ala Leu Ser Leu Val Asp Gln Val Tyr Phe Ile Val Gly
            500                 505                 510

Tyr Ala Ala Leu Asn Leu Cys Asp Phe Asn Leu Met Asp Pro Leu Ile
        515                 520                 525

Glu Gln Val Gln Met Phe Leu Leu His Leu Ser Pro Asn Glu Asp His
    530                 535                 540

Ile Ala Tyr Arg Phe Ser Cys Met Val Ala Glu Phe Lys Arg Arg Cys
545                 550                 555                 560

Gly Ser Ala Glu Cys Asn Asp Pro Ser Ser Thr Val Lys Gly Ser Pro
                565                 570                 575

Leu Ser Ser Tyr Gly Asp Ser Arg Lys Met Ser Met Gly Gln Ala Pro
            580                 585                 590

Phe Met Pro Pro Leu Met Asp Gly Met Ile Glu Gly Tyr Gly Phe Glu
        595                 600                 605

Gln Leu Met Pro Glu Val Met Pro Ser Ser Phe Pro Asp Gly Ile Leu
    610                 615                 620

Asn Gly Met Pro Val Thr Gly Leu Ala Ala Tyr Arg Ser Ala Thr Leu
625                 630                 635                 640

Ser Ser Asn Thr Arg Asp Asp Asn Leu Gln Arg Trp Phe Ser Ser Cys
                645                 650                 655

Pro Trp Ala Glu Leu Lys Pro Arg Thr
            660                 665

<210>  6
<211>  29
<212>  DNA
<213>  Artificial

<220>
<223>  Primer

<400>  6
cggaattcgc atgctggagg tgcttctaa                                          29

<210>  7
<211>  33
<212>  DNA
<213>  Artificial

<220>
<223>  Primer

<400>  7
ttcccaggat tgaggcattt tgaccacgag att                                     33

<210>  8
<211>  17
<212>  DNA
<213>  Artificial

<220>
<223>  Primer

<400>  8
taacttccac cgaggtc                                                       17

<210>  9
<211>  18
<212>  DNA
<213>  Artificial

<220>
<223>  Primer

<400>  9
cgatcgatga ctgcctgt                                                      18

<210>  10
<211>  18
<212>  DNA
<213>  Artificial

<220>
<223>  Primer

<400>  10
agagacacat agtgcctt                                                    18


<210>  11
<211>  18
<212>  DNA
<213>  Artificial

<220>
<223>  Primer

<400>  11
gcttatagtc gatagcgc                                                    18


<210>  12
<211>  18
<212>  DNA
<213>  Artificial

<220>
<223>  Primer

<400>  12
cctctctcca gcgatggt                                                    18


<210>  13
<211>  18
<212>  DNA
<213>  Artificial

<220>
<223>  Primer

<400>  13
atggaataca tactgctt                                                    18


<210>  14
<211>  18
<212>  DNA
<213>  Artificial

<220>
<223>  Primer

<400>  14
atgaaaccca ctgtagct                                                    18


<210>  15

<211>  18
<212>  DNA
<213>  Artificial

<220>
<223>  Primer

<400>  15
tgctcgataa gcgggtcc                                                     18


<210>  16
<211>  18
<212>  DNA
<213>  Artificial

<220>
<223>  Primer

<400>  16
aatcttatgg acccgctt                                                     18


<210>  17
<211>  20
<212>  DNA
<213>  Artificial

<220>
<223>  Primer

<400>  17
ccccgggaaa caagaacagg                                                   20


<210>  18
<211>  20
<212>  DNA
<213>  Artificial

<220>
<223>  Primer

<400>  18
gttggcggac cttgactatg                                                   20


<210>  19
<211>  22
<212>  DNA
<213>  Artificial

<220>
<223>  Primer

<400>  19
acagctacag tgggtttcat ct                                                22


<210>  20
<211>  19

<212>  DNA
<213>  Artificial

<220>
<223>  Primer

<400>  20
agtcaacggg ggaagtctc                                                    19


<210>  21
<211>  20
<212>  DNA
<213>  Artificial

<220>
<223>  Primer

<400>  21
ctagcagcgt atcggtcagc                                                   20


<210>  22
<211>  19
<212>  DNA
<213>  Artificial

<220>
<223>  Primer

<400>  22
cttggaaaag aaacgatag                                                    19


<210>  23
<211>  19
<212>  DNA
<213>  Artificial

<220>
<223>  Primer

<400>  23
aacgtacgct ttcctcctt                                                    19


<210>  24
<211>  21
<212>  DNA
<213>  Artificial

<220>
<223>  Primer

<400>  24
gggtccgtcc agtccgttct t                                                 21


<210>  25
<211>  24
<212>  DNA

<213>  Artificial

<220>
<223>  Primer

<400>  25
agcggataac aatttcacac agga                                              24


<210>  26
<211>  17
<212>  DNA
<213>  Artificial

<220>
<223>  Primer

<400>  26
gttttcccag tcacgac                                                      17


<210>  27
<211>  19
<212>  DNA
<213>  Artificial

<220>
<223>  Primer

<400>  27
tcatccctgg tgttactgc                                                    19


<210>  28
<211>  17
<212>  DNA
<213>  Artificial

<220>
<223>  Primer

<400>  28
catggattgg ctggccg                                                      17


<210>  29
<211>  18
<212>  DNA
<213>  Artificial

<220>
<223>  Primer

<400>  29
tactctccag attgcctg                                                     18


<210>  30
<211>  18
<212>  DNA
<213>  Artificial

<220>
<223>  Primer

<400>  30
tgagatacca ctcagcag                                                    18


<210>  31
<211>  18
<212>  DNA
<213>  Artificial

<220>
<223>  Primer

<400>  31
tgcacttctc tgtctctg                                                    18


<210>  32
<211>  18
<212>  DNA
<213>  Artificial

<220>
<223>  Primer

<400>  32
gacttctggc atcagttg                                                    18


<210>  33
<211>  18
<212>  DNA
<213>  Artificial

<220>
<223>  Primer

<400>  33
ctcatggatg gcatgatc                                                    18

## Description

### Background of the Invention

### Field of the Invention

The present invention relates to isolated nucleic acid sequences encoding polypeptides having transcriptional activation activity and to the polypeptides. The invention also relates to nucleic acid constructs, vectors and host cells comprising the nucleic acid sequences. The invention further relates to host cells useful for the production of polypeptides in which the production or function of the transcriptional activator has been altered, as well as to methods for producing the polypeptides.0

### Description of the Related Art

The use of recombinant host cells in the expression of heterologous proteins has in recent years greatly simplified the production of large quantities of commercially valuable proteins which otherwise are obtainable only by purification from their native sources. Currently, there is a varied selection of expression systems from which to choose for the production of any given protein, including eubacterial and eukaryotic hosts. The selection of an appropriate expression system often depends not only on the ability of the host cell to produce adequate yields of the protein in an active state, but, to a large extent, may also be governed by the intended end use of the protein.

One problem frequently encountered is the high level of proteolytic enzymes produced by a given host cell or present in the culture medium. One suggestion has been to provide host organisms deprived of the ability to produce specific proteolytic compounds. For example, WO 90/00192 (Genencor, Inc.) describes filamentous fungal hosts incapable of secreting enzymatically active aspartic proteinase. EP 574 347 (Ciba Geigy AG) describes *Aspergillus* hosts defective in a serine protease of the subtilisin-type. WO 98/12300 (Novo Nordisk A/S) describes hosts defective in a metalloprotease and an alkaline protease. WO 97/12045 (Genencor, Inc.) describes yeast and bacterial host systems which are rendered protease deficient resulting from a disruption of a promoter sequence involved in the regulation of a protease gene.

Mattern, I.E., et al., (1992. Mol Gen Genet 234:332-336) describe a mutant strain of *Aspergillus niger,* which was shown to have only 1 to 2% of the extracellular protease activity of the parent strain, apparently due to a deficiency of at least two proteases, aspergillopepsin A and aspergillopepsin B. It was suggested that the protease deficient phenotype could result from a regulatory mutation affecting the expression of the genes coding for both proteases.

The initiation of eukaryotic transcription at a specific promoter or set of promoters requires a eukaryotic transcriptional activator which is a polypeptide, but which is not itelf part of RNA polymerase. Many transcriptional activators bind to a specific site on the promoter to form a functional promoter necessary for the initiation of transcription of the polypeptide encoding sequence. However, a transcriptional activator may also be incorporated into an initiation complex only in the presence of other polypeptides. Polypeptides with transcriptional activation activity have been described in fungi, and a list of such polypeptides has been published (Dhawale, S.S., and Lane, A.C. 1993. Nucleic Acid Research 21:5537-5546).

### Solution proposed by the invention:

It is an object of the present invention to provide improved methods for increasing production of polypeptides in host cells in which the activity of a transcriptional activator involved in the regulation of protease production has been modified.

### Summary of the Invention

A first aspect of the present invention relates to an isolated nucleic acid sequence encoding a transcriptional activator selected from the group consisting of:
(a) a nucleic acid sequence having at least 70% identity with the nucleic acid sequence of SEQ ID NO:1;
(b) a nucleic acid sequence encoding a polypeptide having an amino acid sequence which has at least 50% identity with the amino acid sequence of SEQ ID NO:2;
(c) a nucleic acid sequence which hybridizes under low stringency conditions with (i) the nucleic acid sequence of SEQ ID NO:1, or (ii) its complementary strand, wherein the low stringency conditions are defined by prehybridization and hybridization at 42°C in 5x SSPE, 0.3% SDS, 200 mg/ml sheared and denatured salmon sperm DNA, and 25% formamide, and wash conditions are defined by 50°C for 30 minutes in 2X SSC, 0.2% SDS;
(d) an allelic variant of (a), (b), or (c);
(e) a subsequence of (a), (b), (c), or (d), wherein the subsequence encodes a polypeptide fragment which has transcriptional activation activity; and
(f) a subsequence of (a), (b) (c), or (d), wherein the subsequence encodes a polypeptide with the amino acid sequence of SEQ ID NO:3.

In another aspect, the invention also relates to nucleic acid constructs, vectors and host cells comprising the nucleic acid sequences, and to the polypeptides encoded by the nucleic acid sequences. The invention further relates to host cells useful for the production of a polypeptide, in which the production or function of the transcriptional activator has been altered, as well as to methods for producing the polypeptide.

### Brief Description of the Figures

Figure 1 shows a restriction map of the plasmid pPAP, the construction of which is described in Example 1.
Figure 2 shows a restriction map of the plasmid pAopyrGcosArp1, the construction of which is described in Example 1.
Figure 3 shows a restriction map of the plasmid pEES1, the construction of which is described in Example 1.
Figure 4 shows a restriction map of the plasmid pDprt, the construction of which is described in Example 3.
Figure 5 shows a restriction map of the plasmid pGPprt, the construction of which is described in Example 4.

### Detailed Description of the Invention

### Nucleic Acid Sequences Encoding Transcriptional Activators

A first aspect of the present invention relates to an isolated nucleic acid sequence encoding a transcriptional activator selected from the group consisting of:
(a) a nucleic acid sequence having at least 70% identity with the nucleic acid sequence of SEQ ID NO:1;
(b) a nucleic acid sequence encoding a polypeptide having an amino acid sequence which has at least 50% identity with the amino acid sequence of SEQ ID NO:2;
(c) a nucleic acid sequence which hybridizes under low stringency conditions with (i) the nucleic acid sequence of SEQ ID NO:1, or (ii) its complementary strand, wherein the low stringency conditions are defined by prehybridization and hybridization at 42°C in 5x SSPE, 0.3% SDS, 200 mg/ml sheared and denatured salmon sperm DNA, and 25% formamide, and wash conditions are defined by 50°C for 30 minutes in 2X SSC, 0.2% SDS;
(d) an allelic variant of (a), (b), or (c);
(e) a subsequence of (a), (b), (c), or (d), wherein the subsequence encodes a polypeptide fragment which has transcriptional activation activity; and
(f) a subsequence of (a), (b), (c), or (d), wherein the subsequence encodes a polypeptide with the amino acid sequence of SEQ ID NO:3.

The term "transcriptional activator" as used herein refers to a polypeptide which has the capability to activate a specific promoter or set of promoters necessary for the initiation of transcription of the polypeptide encoding sequence to which it is linked.

The term "isolated nucleic acid sequence" as used herein refers to a nucleic acid sequence which is essentially free of other nucleic acid sequences, *e.g.*, at least about 20% pure, preferably at least about 40% pure, more preferably at least about 60% pure, even more preferably at least about 80% pure, most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

In a preferred embodiment, the nucleic acid sequence has a degree of identity to the nucleic acid sequence set forth in SEQ ID NO:1 of at least about 70%, preferably at least about 80%, more preferably at least about 85%, even more preferably at least about 90%, most preferably at least about 95%, and even most preferably at least about 97% identity, which encodes an active polypeptide. For purposes of the present invention, the degree of identity between two nucleic acid sequences is determined by the Clustal method (Higgins, 1989, CABIOS 5:151-153) with an identity table, a gap penalty of 10, and a gap length penalty of 10.

In an even more preferred embodiment, the nucleic acid sequence encoding a transcriptional activator has a nucleic acid sequence as set forth in SEQ ID NO:1.

Modification of a nucleic acid sequence encoding a polypeptide of the present invention may be necessary for the synthesis of polypeptides substantially similar to the polypeptide. The term "substantially similar" to the polypeptide refers to non-naturally occurring forms of the polypeptide. These polypeptides may differ in some engineered way from the polypeptide isolated from its native source. For example, it may be of interest to synthesize variants of the polypeptide where the variants differ in specific activity, binding specificity and/or affinity, or the like using, *e.g.*, site-directed mutagenesis. The analogous sequence may be constructed on the basis of the nucleic acid sequence presented as the polypeptide encoding part of SEQ ID NO:1, *e.g.,* a subsequence thereof, and/or by introduction of nucleotide substitutions which do not give rise to another amino acid sequence of the polypeptide encoded by the nucleic acid sequence, but which corresponds to the codon usage of the host organism intended for production of the enzyme, or by introduction of nucleotide substitutions which may give rise to a different amino acid sequence. For a general description of nucleotide substitution, see, *e.g.*, Ford et al., 1991, Protein Expression and Purification 2: 95-107.

In another preferred embodiment, the present invention relates to isolated nucleic acid sequences encoding polypeptides having an amino acid sequence which has a degree of identity to the amino acid sequence set forth in SEQ ID NO:2 of at least about 50%, preferably at least about 60%, preferably at least about 70%, more preferably at least about 80%, even more preferably at least about 90%, most preferably at least about 95%, and even most preferably at least about 97%, which qualitatively retain the transcriptional activation activity of the polypeptides (hereinafter "homologous polypeptides").

In a preferred embodiment, the homologous polypeptides have an amino acid sequence which differs by five amino acids, preferably by four amino acids, more preferably by three amino acids, even more preferably by two amino acids, and most preferably by one amino acid from the amino acid sequence set forth in SEQ ID NO:2. For purposes of the present invention, the degree of identity between two amino acid sequences is determined by the Clustal method (Higgins, 1989, *supra)* with an identity table, a gap penalty of 10, and a gap length penalty of 10.

Hybridization indicates that by methods of standard Southern blotting procedures, the nucleic acid sequence hybridizes to an oligonucleotide probe corresponding to the polypeptide encoding part of the nucleic acid sequence shown in SEQ ID NO:1, under low to high stringency conditions (*i.e.*, prehybridization and hybridization at 42°C in 5x SSPE, 0.3% SDS, 200 µg/ml sheared and denatured salmon sperm DNA, and either 25, 35 or 50% formamide for low, medium and high stringencies, respectively). In order to identify a clone or DNA which is homologous with SEQ ID NO:1, the hybridization reaction is washed three times for 30 minutes each using 2X SSC, 0.2% SDS preferably at least 50°C, more preferably at least 55°C, more preferably at least 60°C, more preferably at least 65°C, even more preferably at least 70°C, and most preferably at least 75°C.

The nucleic acid sequence of SEQ ID NO:1, or a subsequence thereof, as well as the amino acid sequence of SEQ ID NO:2, or a partial sequence thereof, or the amino acid sequence of SEQ ID NO:3, may be used to design an oligonucleotide probe to identify and isolate or clone a homologous gene of any genus or species according to methods well known in the art.

In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 40 nucleotides in length. Longer probes can also be used. Both DNA and RNA probes can be used. The probes are typically labeled for detecting the corresponding gene (for example, with ³²P, ³H, ³⁵S, biotin, or avidin). For example, molecules to which a ³²P-, ³H- or ³⁵S-labelled oligonucleotide probe hybridizes may be detected by use of X-ray film.

Thus, a genomic, cDNA or combinatorial chemical library prepared from such other organisms may be screened for DNA which hybridizes with the probes described above and which encodes a polypeptide with transcriptional activation activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. A clone or DNA which is homologous to SEQ ID NO:1 may then be identified following standard Southern blotting procedures.

An allelic variant denotes any of two or more alternative forms of a gene occupying the same chomosomal locus. Allelic variation arises naturally through mutation, and may result in phenotypic polymorphism within populations. Gene mutations can be silent (*i.e.*, no change in the encoded polypeptide) or may encode polypeptides having altered amino acid sequences.

The term "allelic variant of a polypeptide" is a polypeptide encoded by an allelic variant of a gene. In a preferred embodiment, the nucleic acid sequence encoding a transcriptional activator of the present invention is an allelic variant of a nucleic acid sequence selected from the group consisting of nucleic acid sequences: (a) having at least 70% identity with the nucleic acid sequence of SEQ ID NO:1, (b) encoding a polypeptide having an amino acid sequence which has at least 50% identity with the amino acid sequence of SEQ ID NO:2, (c) which hybridizes under low stringency conditions with the nucleic acid sequence of SEQ ID NO:1, or its complementary strand, and (d) encoding a polypeptide having the amino acid sequence of SEQ ID NO:3.

The present invention also encompasses nucleic acid sequences which differ from SEQ ID NO:1 by virtue of the degeneracy of the genetic code. The present invention also relates to subsequences of SEQ ID NO:1, wherein a subsequence of SEQ ID NO:1 is a nucleic acid sequence encompassed by SEQ ID NO:1 except that one or more nucleotides from the 5' and/or 3' end have been deleted. Preferably, a subsequence of SEQ ID NO:1 encodes a polypeptide fragment which has transcriptional activation activity. In a more preferred embodiment, a subsequence of SEQ ID NO:1 contains at least a nucleic acid sequence encoding the polypeptide sequence shown in SEQ ID NO:3.

The techniques used to isolate or clone a nucleic acid sequence encoding a polypeptide are known in the art and include isolation from genomic DNA, preparation from cDNA, or a combination thereof. The cloning of the nucleic acid sequences of the present invention from such genomic DNA can be effected, *e.g.*, by using methods based on polymerase chain reaction (PCR) or antibody screening of expression libraries to detect cloned DNA fragments with shared structural features. (See, *e.g.*, Innis et al., 1990, PCR: A Guide to Methods and Application, Academic Press, New York.) Other nucleic acid amplification procedures such as ligase chain reaction (LCR), ligated activated transcription (LAT) and nucleic acid sequence-based amplification (NASBA) may be used. The nucleic acid sequence may be cloned from a microorganism, or another or related organism and thus, for example, may be an allelic or species variant of the polypeptide encoding region of the nucleic acid sequence.

The transcriptional activators encoded by nucleic acid sequences which hybridize with an oligonucleotide probe which hybridizes with the nucleic acid sequence of SEQ ID NO:1, its complementary strand, or allelic variants and subsequences of SEQ ID NO:1, or allelic variants and fragments of the transcriptional activators may be obtained from microorganisms of any genus.

In a preferred embodiment, the transcriptional activators may be obtained from a fungal cell. "Fungi" as used herein includes the phyla Ascomycota, Basidiomycota, Chytridiomycota, and Zygomycota (as defined by Hawksworth, et al., in Ainsworth and Bisby's Dictionary of The Fungi, 8th edition, 1995, CAB International, University Press, Cambridge, UK) as well as the Oomycota (as cited in Hawksworth *et al*., 1995, *supra,* page 171) and all mitosporic fungi (Hawksworth *et al*., 1995, *supra).*

In preferred embodiment, the fungal source is a filamentous fungal strain. "Filamentous fungi" include all filamentous forms of the subdivision Eumycota and Oomycota (as defined by Hawksworth *et al*., 1995, *supra).* The filamentous fungi are characterized by a mycelial wall composed of chitin, cellulose, glucan, chitosan, mannan, and other complex polysaccharides. Vegetative growth is by hyphal elongation and carbon catabolism is obligately aerobic. Filamentous fungal strains include, but are not limited to, strains of *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium,* and *Trichoderma.*

In a more preferred embodiment, the nucleic acid sequence encoding a transcriptional activator of the present invention is obtained from a strain of *Aspergillus,* such as *A. awamori* or *A. nidulans.* Preferably, the nucleic acid sequence is obtained from a strain of *A. niger* or *A. oryzae.* Even more preferably, the nucleic acid sequence is obtained from an isolate of a strain of *A. niger,* DSM 12298; e.g., the nucleic acid sequence set forth in SEQ ID NO:1.

In another more preferred embodiment, the nucleic acid sequence encoding a transcriptional activator of the present invention is obtained from a strain of *Fusarium,* such as *F. oxysporum.* Preferably, the strain is a strain of *F. venenatum* (Nirenberg sp. nov.).

In another preferred embodiment, the nucleic acid sequence encoding a transcriptional activator of the present invention is obtained from a yeast strain, such as a *Candida, Kluyveromyces, Schizosaccharomyces,* or *Yarrowia* strain. Preferably, the strain is a strain of *Hansenula, Pichia,* or *Saccharomyces.*

It will be understood that for the aforementioned species, the invention encompasses both the perfect and imperfect states, and other taxonomic equivalents, *e.g.*, anamorphs, regardless of the species name by which they are known. Those skilled in the art will readily recognize the identity of appropriate equivalents. For example, the polypeptides may be obtained from microorganisms which are taxonomic equivalents of *Aspergillus* as defined by Raper, K.D. and Fennel, D.I. (1965. *The Genus Aspergillus,* The Wilkins Company, Baltimore MD). regardless of the species name by which they are known. *Aspergilli* are mitosporic fungi characterized by an aspergillum comprised of a conidiospore stipe with no known teleomorphic states terminating in a vesicle, which in turn bears one or two layers of synchronously formed specialized cells, variously referred to as sterigmata or phialides, and asexually formed spores referred to as conidia. Known teleomorphs of *Aspergillus* include *Eurotium, Neosartorya,* and *Emericella.* Strains of *Aspergillus* and teleomorphs thereof are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Furthermore, such transcriptional activators may be identified and obtained from other sources including microorganisms isolated from nature (*e.g.*, soil, composts, water, etc.) using the above-mentioned probes. Techniques for isolating microorganisms from natural habitats are well known in the art. The nucleic acid sequence may then be derived by similarly screening a genomic or cDNA library of another microorganism. Once a nucleic acid sequence encoding a transcriptional activator has been detected with the probe(s), the sequence may be isolated or cloned by utilizing techniques which are known to those of ordinary skill in the art (see, *e.g.,* J. Sambrook, E.F. Fritsch, and T. Maniatus, 1989, Molecular Cloning, A Laboratory Manual, 2d edition, Cold Spring Harbor, New York).

In another preferred embodiment, the isolated nucleic acid sequence encodes a polypeptide comprising the amino acid sequence of SEQ ID NO:2, or a fragment thereof, which has transcriptional activation activity.

In another preferred embodiment, the isolated nucleic acid sequence encodes a polypeptide comprising the amino acid sequence of SEQ ID NO:3.

The present invention also relates to isolated nucleic acid sequences encoding a transcriptional activator of the present invention, which, *e.g.*, using methods of standard Southern blotting procedures described above (*cf.,* Sambrook, *et al.,* 1989, *supra),* hybridize under low stringency conditions, more preferably medium stringency conditions, and most preferably high stringency conditions, with an oligonucleotide probe which hybridizes under the same conditions with the nucleic acid sequence set forth in SEQ ID NO:1 or its complementary strand, or allelic variants and subsequences of SEQ ID NO:1 which encode polypeptide fragments which are transcriptional activators in fungi.

In another more preferred embodiment, the nucleic acid sequence is the nucleic acid sequence encoding a polypeptide which has DNA binding activity contained in the plasmid pEES which is contained in *Escherichia coli* DSM 12294.

### Nucleic Acid Constructs

Another aspect of the present invention relates to nucleic acid constructs comprising a nucleic acid sequence encoding a transcriptional activator of the present invention operably linked to one or more control sequences which direct the production of the transcriptional activator in a suitable expression host. In a preferred embodiment, the nucleic acid sequence encodes a polypeptide which is contained in the plasmid pEES harboured in *Escherichia coli* DSM 12294.

Expression will be understood to include any step involved in the production of the polypeptide including, but not limited to, transcription, post-transcriptional modification, translation, post-translational modification, and secretion. Manipulation of the nucleic acid sequence encoding a polypeptide prior to its insertion into a vector may be desirable or necessary depending on the expression vector. The techniques for modifying nucleic acid sequences utilizing cloning methods are well known in the art.

"Nucleic acid construct" is defined herein as a nucleic acid molecule, either single- or double-stranded, which is isolated from a naturally occurring gene or which has been modified to contain segments of nucleic acid which are combined and juxtaposed in a manner which would not otherwise exist in nature. The term nucleic acid construct is synonymous with the term expression cassette when the nucleic acid construct contains all the control sequences required for expression of a coding sequence. The term "coding sequence" as defined herein is a sequence which is transcribed into mRNA and translated into a transcriptional avtivator of the present invention. The boundaries of the coding sequence are generally determined by the ATG start codon at the 5' end of the mRNA and a transcription terminator sequence located just downstream of the open reading frame at the 3' end of the mRNA. A coding sequence can include, but is not limited to, DNA, cDNA, and recombinant nucleic acid sequences.

The term "control sequences" is defined herein to include all components which are necessary or advantageous for the expression of a polypeptide. Each control sequence may be native or foreign to the nucleic acid sequence encoding the polypeptide. Such control sequences include, but are not limited to, a leader, a polyadenylation sequence, a propeptide sequence, a promoter, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide. The term "operably linked" is defined herein as a configuration in which a control sequence is appropriately placed at a position relative to the coding sequence of the DNA sequence such that the control sequence directs the production of a polypeptide.

The control sequence may be an appropriate promoter sequence, a nucleic acid sequence which is recognized by a host cell for expression of the nucleic acid sequence. The promoter sequence contains transcriptional control sequences which mediate the expression of the polypeptide. The promoter may be any nucleic acid sequence which shows transcriptional activity in the cell including mutant, truncated, and hybrid promoters, and may be obtained from genes encoding extracellular or intracellular polypeptides either homologous or heterologous to the cell.

The control sequence may also be a suitable transcription terminator sequence, a sequence recognized by a filamentous fungal cell to terminate transcription. The terminator sequence is operably linked to the 3' terminus of the nucleic acid sequence encoding the polypeptide. Any terminator which is functional in the cell may be used in the present invention.

Preferred terminators for filamentous fungal cells are obtained from the genes encoding *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

The control sequence may also be a suitable leader sequence, a nontranslated region of a mRNA which is important for translation by the filamentous fungal cell. The leader sequence is operably linked to the 5' terminus of the nucleic acid sequence encoding the polypeptide. Any leader sequence which is functional in the cell may be used in the present invention.

Preferred leaders for filamentous fungal cells are obtained from the genes encoding *Aspergillus oryzae* TAKA amylase and *Aspergillus nidulans* triose phosphate isomerase.

The control sequence may also be a polyadenylation sequence, a sequence which is operably linked to the 3' terminus of the nucleic acid sequence and which, when transcribed, is recognized by the filamentous fungal cell as a signal to add polyadenosine residues to transcribed mRNA. Any polyadenylation sequence which is functional in the cell may be used in the present invention.

Preferred polyadenylation sequences for filamentous fungal cells are obtained from the genes encoding *Aspergillus oryzae* TAKA amylase, *Aspergillus niger* glucoamylase, *Aspergillus nidulans* anthranilate synthase, and *Aspergillus niger* alpha-glucosidase.

The control sequence may also be a signal peptide coding region, which codes for an amino acid sequence linked to the amino terminus of the polypeptide which can direct the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not normally contain a signal peptide coding region. Alternatively, the foreign signal peptide coding region may simply replace the natural signal peptide coding region in order to obtain enhanced secretion of the polypeptide. The signal peptide coding region may be obtained from a glucoamylase or an amylase gene from an *Aspergillus* species, or a lipase or proteinase gene from a *Rhizomucor* species. However, any signal peptide coding region which directs the expressed polypeptide into the secretory pathway of a filamentous fungal cell may be used in the present invention.

An effective signal peptide coding region for filamentous fungal cells is the signal peptide coding region obtained from the *Aspergillus oryzae* TAKA amylase gene, *Aspergillus niger* neutral amylase gene, *Rhizomucor miehei* aspartic proteinase gene, or *Humicola lanuginosa* cellulase gene.

The control sequence may also be a propeptide coding region, which codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the *Rhizomucor miehei* aspartic proteinase gene, or the *Myceliophthora thermophila* laccase gene (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

### Expression Vectors

The present invention also relates to recombinant expression vectors comprising a nucleic acid sequence of the present invention, a promoter, and transcriptional and translational stop signals. The various nucleic acid and control sequences described above may be joined together to produce a recombinant expression vector which may include one or more convenient restriction sites to allow for insertion or substitution of the nucleic acid sequence encoding the polypeptide at such sites. Alternatively, the nucleic acid sequence encoding the polypeptide may be expressed by inserting the sequence or a nucleic acid construct comprising the sequence into an appropriate vector for expression. In creating the expression vector, the coding sequence is located in the vector so that the coding sequence is operably linked with the appropriate control sequences for expression, and possibly secretion.

The recombinant expression vector may be any vector (*e.g.*, a plasmid or virus) which can be conveniently subjected to recombinant DNA procedures and can bring about the expression of the nucleic acid sequence encoding the polypeptide. The choice of the vector will typically depend on the compatibility of the vector with the filamentous fungal cell into which the vector is to be introduced. The vectors may be linear or closed circular plasmids. The vector may be an autonomously replicating vector, *i.e.*, a vector which exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one which, when introduced into the filamentous fungal cell, is integrated into the genome and replicated together with the chromosome(s) into which it has been integrated. The vector system may be a single vector or plasmid or two or more vectors or plasmids which together contain the total DNA to be introduced into the genome of the filamentous fungal cell, or a transposon.

The vectors preferably contain one or more selectable markers which permit easy selection of transformed cells. A selectable marker is a gene the product of which provides for biocide or viral resistance, resistance to heavy metals, prototrophy to auxotrophs, and the like. A selectable marker for use in a filamentous fungal cell may be selected from the group including, but not limited to, *amdS* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents from other species. Preferred for use in an *Aspergillus* cell are the *amds* and *pyrG* genes of *Aspergillus nidulans* or *Aspergillus oryzae* and the bar gene of *Streptomyces hygroscopicus.*

The vectors preferably contain an element(s) that permits stable integration of the vector into the host cell genome or autonomous replication of the vector in the cell independent of the genome of the cell.

### Host Cells

Another aspect of the present invention relates to host cells comprising a nucleic acid construct or an expression vector of the present invention.

The choice of a host cell in the methods of the present invention will to a large extent depend upon the source of the nucleic acid sequence encoding the polypeptide of interest.

The introduction of an expression vector or a nucleic acid construct into a filamentous fungal cell may involve a process consisting of protoplast formation, transformation of the protoplasts, and regeneration of the cell wall in a manner known *per se.* Suitable procedures for transformation of *Aspergillus* cells are described in EP 238 023 and Yelton et al., 1984, Proceedings of the National Academy of Sciences USA 81: 1470-1474. A suitable method of transforming *Fusarium* species is described by Malardier et al., 1989, Gene 78: 147-156 or in WO 96/00787.

"Introduction" means introducing a vector comprising the nucleic acid sequence encoding the polypeptide into a filamentous fungal cell so that the vector is maintained as a chromosomal integrant or as a self-replicating extrachromosomal vector. Integration is generally considered to be an advantage as the nucleic acid sequence is more likely to be stably maintained in the cell. Integration of the vector into the chromosome occurs by homologous recombination, nonhomologous recombination, or transposition.

For integration into the host cell genome, the vector may rely on the nucleic acid sequence encoding the polypeptide or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. These nucleic acid sequences may be any sequence that is homologous with a target sequence in the genome of the host cell, and, furthermore, may be non-encoding or encoding sequences.

For autonomous replication, the vector may further comprise an origin of replication enabling the vector to replicate autonomously in the host cell.

The procedures used to ligate the elements described above to construct the recombinant expression vectors are well known to one skilled in the art (see, *e.g.*, Sambrook, *et al., supra).*

In a preferred embodiment, the filamentous fungal host cell is a cell of a species of, but not limited to, *Acremonium, Aspergillus, Fusarium, Humicola, Mucor, Myceliophthora, Neurospora, Penicillium, Thielavia, Tolypocladium,* or *Trichoderma.*

In a more preferred embodiment, the filamentous fungal cell is an *Aspergillus* cell. In another more preferred embodiment, the filamentous fungal cell is an *Acremonium* cell. In another more preferred embodiment, the filamentous fungal cell is a *Fusarium* cell. In another more preferred embodiment, the filamentous fungal cell is a *Humicola* cell. In another more preferred embodiment, the filamentous fungal cell is a *Mucor* cell. In another more preferred embodiment, the filamentous fungal cell is a *Myceliophthora* cell. In another more preferred embodiment, the filamentous fungal cell is a *Neurospora* cell. In another more preferred embodiment, the filamentous fungal cell is a *Penicillium* cell. In another more preferred embodiment, the filamentous fungal cell is a *Thielavia* cell. In another more preferred embodiment, the filamentous fungal cell is a *Tolypocladium* cell. In another more preferred embodiment, the filamentous fungal cell is a *Trichoderma* cell.

In a most preferred embodiment, the filamentous fungal cell is an *Aspergillus awamori, Aspergillus foetidus, Aspergillus japonicus, Aspergillus nidulans, Aspergillus niger* or *Aspergillus oryzae* cell. In another most preferred embodiment, the filamentous fungal cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sulphureum, Fusarium toruloseum, Fusarium trichothecioides,* or *Fusarium venenatum* cell. In an even most preferred embodiment, the filamentous fungal cell is a *Fusarium venenatum* (Nirenberg sp. nov.). In another most preferred embodiment, the filamentous fungal cell is a *Humicola insolens* or *Humicola lanuginosa* cell. In another most preferred embodiment, the filamentous fungal cell is a *Mucor miehei* cell. In another most preferred embodiment, the filamentous fungal cell is a *Myceliophthora thermophilum* cell. In another most preferred embodiment, the filamentous fungal cell is a *Neurospora crassa* cell. In another most preferred embodiment, the filamentous fungal cell is a *Penicillium purpurogenum* cell. In another most preferred embodiment, the filamentous fungal cell is a *Thielavia terrestris* cell. In another most preferred embodiment, the *Trichoderma* cell is a *Trichoderma harzianum, Trichoderma koningii, Trichoderma longibrachiatum, Trichoderma reesei* or *Trichoderma viride* cell.

### Polypeptides having Transcriptional Activation Activity

Another aspect of the present invention relates to an isolated polypeptide selected from the group consisting of:
(a) a polypeptide which is encoded in a nucleic acid sequence which hybridizes under low stringency conditions with (i) the nucleic acid sequence of SEQ ID NO:1; (ii) its complementary strand, or (iii) a subsequence of SEQ ID NO:1 which encodes a polypeptide fragment which has transcriptional activation activity, wherein the low stringency conditions are defined by prehybridization and hybridization at 42°C in 5X SSPE, 0.3% SDS, 200 µg/ml sheared and denatured salmon sperm DNA, and 25% formamide, and wash conditions are defined at 50°C for 30 minutes in 2X SSC, 0.2% SDS;
(b) a polypeptide having an amino acid sequence which has at least 50% identity with the amino acid sequence of SEQ ID NO:2;
(c) an allelic variant of (a) or (b);
(d) a fragment of (a), (b), or (c), wherein the fragment has transcriptional activation activity; and
(e) a polypeptide comprising the amino acid sequence of SEQ ID NO:3, or an allelic variant thereof.

The transcriptional activator may be isolated using techniques as described herein. As defined herein, an "isolated" polypeptide is a polypeptide which is essentially free of other polypeptides, *e.g.*, at least about 20% pure, preferably at least about 40% pure, more preferably about 60% pure, even more preferably about 80% pure, most preferably about 90% pure, and even most preferably about 95% pure, as determined by SDS-PAGE.

The present invention also relates to isolated polypeptides having an amino acid sequence which has a degree of identity to the amino acid sequence of SEQ ID NO:2 of at least about 50%, preferably at least about 55%, preferably at least about 60%, preferably at least about 65%, preferably at least about 70%, preferably at least about 75%, preferably at least about 80%, more preferably at least about 85%, even more preferably at least about 90%, most preferably at least about 95%, and even most preferably at least about 97%, which have transcriptional activation activity.

In more preferred embodiment, the transcriptional activator of the present invention comprises the amino acid sequence of SEQ ID NO:2, or a fragment thereof, wherein the fragment retains transcriptional activation activity. In a most preferred embodiment, the polypeptide has the amino acid sequence of SEQ ID NO:2. A fragment of SEQ ID NO:2 is a polypeptide having one or more amino acids deleted from the amino and/or carboxy terminus of this amino acid sequence. Preferably, a fragment of SEQ ID NO:2 contains at least the polypeptide sequence shown in SEQ ID NO:3.

The amino acid sequences of the homologous polypeptides may differ from the amino acid sequence of SEQ ID NO:2 or SEQ ID NO:3 by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (such as arginine, lysine and histidine), acidic amino acids (such as glutamic acid and aspartic acid), polar amino acids (such as glutamine and asparagine), hydrophobic amino acids (such as leucine, isoleucine and valine), aromatic amino acids (such as phenylalanine, tryptophan and tyrosine), and small amino acids (such as glycine, alanine, serine, threonine and methionine). Amino acid substitutions which do not generally alter the specific activity are known in the art and are described, for example, by H. Neurath and R.L. Hill (1979. The Proteins, Academic Press, New York). The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

In a more preferred embodiment, a transcriptional activator of the present invention is obtained from an *Aspergillus niger* strain, more preferably from *Aspergillus niger* AB4.1 (van Haringsveldt, W., et al., 1987. Mol. Gen. Genet. 206:71-75), and most preferably from *Aspergillus niger* 13PAP2, which has been deposited at DSM as DSM 12298, or a mutant strain thereof, harbouring, *e.g.*, the polypeptide with the amino acid sequence of SEQ ID NO:2 or SEQ ID NO:3.

In another preferred embodiment, the transcriptional activator of the present invention is the polypeptide encoded in the nucleic acid sequence contained in plasmid pEES which is contained in *Escherichia coli* DSM 12294.

The present invention further relates to methods for producing the transcriptional activator of the present invention comprising (a) cultivating a host cell harbouring a nucleic acid construct or an expression vector comprising a nucleic acid sequence encoding the transcriptional activator of the invention under conditions conducive for production of the polypeptide; and (b) recovering the polypeptide.

### Host Cells having Altered Transcriptional Activation Activity

Another aspect of the present invention relates to a host cell which is a mutant of a parent fungal cell useful for the production of a polypeptide in which the parent cell comprises one or more nucleic acid sequences encoding a protease, the transcription of which is activated by a transcriptional activator of the present invention, and the mutant cell produces less of the transcriptional activator and the protease(s) than the parent cell when cultured under the same conditions.

The mutant cell may be constructed using methods well known in the art; for example, by one or more nucleotide insertions or deletions of the gene encoding the transcriptional activator.

In a preferred embodiment the mutant cell is obtained by modification or inactivation of a nucleic acid sequence present in the cell and necessary for expression of the transcriptional activator.

In a more preferred embodiment, the nucleic acid sequence is selected from the group consisting of: (a) a nucleic acid sequence having at least 70% identity with the nucleic acid sequence of SEQ ID NO:1; (b) a nucleic acid sequence encoding a polypeptide having an amino acid sequence which has at least 50% identity with the amino acid sequence of SEQ ID NO:2; (c) a nucleic acid sequence which hybridizes under low stringency conditions with (i) the nucleic acid sequence of SEQ ID NO:1, or (ii) its complementary strand, (d) an allelic variant of (a), (b), or (c); (e) a subsequence of (a), (b), (c), or (d), wherein the subsequence encodes a polypeptide fragment which has transcriptional activation activity; and (f) a subsequence of (a), (b) (c), or (d), wherein the subsequence encodes a polypeptide with the amino acid sequence of SEQ ID NO:3.

In another preferred embodiment the reduced expression of the transcriptional activator in the mutant cell is obtained by modification or inactivation of a control sequence required for the expression of the transcriptional activator. The term "control sequence" is defined, *supra,* in the section entitled "Nucleic Acid Constructs." In a more preferred embodiment the control sequence in the mutant cell is a promoter sequence or a functional part thereof, *i.e.*, a part which is sufficient for affecting expression of the nucleic acid sequence. Other control sequences for possible modification include, but are not limited to, a leader, a polyadenylation sequence, a propeptide sequence, a signal sequence, and a transcription terminator.

Modification or inactivation of the gene may be performed by subjecting the parent cell to mutagenesis and selecting for mutant cells in which the capability to produce a transcriptional activator has been reduced. The mutagenesis, which may be specific or random, may be performed, for example, by use of a suitable physical or chemical mutagenizing agent, by use of a suitable oligonucleotide, or by subjecting the DNA sequence to PCR generated mutagenesis. Furthermore, the mutagenesis may be performed by use of any combination of these mutagenizing agents.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues.

When such agents are used, the mutagenesis is typically performed by incubating the parent cell to be mutagenized in the presence of the mutagenizing agent of choice under suitable conditions, and selecting for mutant cells exhibiting reduced expression of the gene.

Modification or inactivation of the gene may be accomplished by introduction, substitution, or removal of one or more nucleotides in the gene's nucleic acid sequence or a regulatory element required for the transcription or translation thereof. For example, nucleotides may be inserted or removed so as to result in the introduction of a stop codon, the removal of the start codon, or a change of the open reading frame. Such modification or inactivation may be accomplished by site-directed mutagenesis or PCR generated mutagenesis in accordance with methods known in the art. Although, in principle, the modification may be performed *in vivo, i.e*., directly on the fungal cell expressing the gene to be modified, it is preferred that the modification be performed *in vitro* as exemplified below.

An example of a convenient way to inactivate or reduce expression of the gene by a fungal cell of choice is based on techniques of gene replacement or gene interruption. For example, in the gene interruption method, a nucleic acid sequence corresponding to the endogenous gene or gene fragment of interest is mutagenized *in vitro* to produce a defective nucleic acid sequence which is then transformed into the parent cell to produce a defective gene. By homologous recombination, the defective nucleic acid sequence replaces the endogenous gene or gene fragment. It may be desirable that the defective gene or gene fragment also encodes a marker which may be used for selection of transformants in which the nucleic acid sequence has been modified or destroyed.

Alternatively, modification or inactivation of the gene may be performed by established anti-sense techniques using a nucleotide sequence complementary to the nucleic acid sequence of the gene. More specifically, expression of the gene by a filamentous fungal cell may be reduced or eliminated by introducing a nucleotide sequence complementary to the nucleic acid sequence which may be transcribed in the cell and is capable of hybridizing to the mRNA produced in the cell. Under conditions allowing the complementary anti-sense nucleotide sequence to hybridize to the mRNA, the amount of protein translated is thus reduced or eliminated.

A nucleic acid sequence complementary to the nucleic acid sequence of SEQ ID NO:1 may be obtained from any microbial source. The preferred sources are fungal sources, *e.g.*, yeast and filamentous fungi as described *supra.* Preferred filamentous fungal sources include, but are not limited to, species of *Acremonium, Aspergillus, Fusarium, Humicola, Myceliophthora, Mucor, Neurospora, Penicillium, Phanerochaete, Thielavia, Tolypocladium,* and *Trichoderma.* Preferred yeast sources include, but are not limited to, species of *Candida, Hansenula, Kluyveromyces, Pichia, Saccharomyces, Schizosaccharomyces,* and *Yarrowia.* Furthermore, the nucleic acid sequence may be native to the filamentous fungal cell.

In another preferred embodiment, the parent cell harbours a gene having a nucleic acid sequence encoding a polypeptide with an amino acid sequence which has at least 50% identity with the amino acid sequence of SEQ ID NO:2.

In another preferred embodiment, the parent cell harbours a gene having a nucleic acid sequence with at least 70% identity with the nucleic acid sequence of SEQ ID NO:1.

In another preferred embodiment, the mutant cell harbours a nucleic acid sequence which has been modified or inactivated by any of the methods described above and produces less of a protease or a combination of proteases than the parent cell when cultured under identical conditions. The mutant cell produces preferably at least about 25% less, more preferably at least about 50% less, even more preferably at least about 75% less, and even more preferably at least about 95% less of a protease or a comination of proteases than the parent cell when cultured under identical conditions.

In an even more preferred embodiment, the mutant cell produces essentially undetectable amounts of a protease or combination of proteases than the parent cell when cultured under identical conditions.

The protease(s) may be assayed using known methods. In one such method, an aliquot of a 48 hour culture media is incubated with ³H-labelled sperm whale myoglobin at pH 4.0 and the radioactivity in the TCA-soluble fraction is measured (van Noort, J.M., et al., 1991. Anal. Biochem 198:385-390). Other methods have been described for identifying, e.g., aspartic proteinase A. of *A. niger* (Takahashi, K., 1991. Meth. in Enzymol. 248:146-155), endopeptidases (Morihara, K., 1995. Meth. in Enzymol. 248:242-253), carboxypeptidases ( Reminton, J., and Breddam, K., 1994. Meth. in Enzymol. 244:231-248), dipeptidyl peptidase (Ikehara, Y., et al., 244:215-227), and aminopeptidases (Little, G., et al., 1976. Meth. in Enzymol. 45:495-503).

In another preferred embodiment, the mutant cell harbours at least one copy of a nucleic acid sequence encoding a polypeptide of interest.

Another aspect of the present invention relates to a host cell useful for the production of a polypeptide wherein the host cell is a mutant of a parent fungal cell in which the mutant (a) produces more of the transcriptional activator of the present invention as compared to the parent cell when cultured under the same conditions; and (b) comprises a DNA sequence encoding the polypeptide, the transcription of which is activated by the transcriptional activator.

In a preferred embodiment, the host cell produces more of the transcriptional activator than the parent cell when cultured under the same conditions by introducing into the parent cell one or more copies of (i) a nucleic acid sequence encoding a transcriptional activator, (ii) a nucleic acid construct comprising a nucleic acid sequence encoding a transcriptional activator, or (iii) an expression vector as defined above in the section "Expression Vectors".

The nucleic acid construct comprising a nucleic acid sequence encoding a transcriptional activator of the present invention may also comprise one or more nucleic acid sequences which encode one or more factors that are advantageous for directing the expression of the polypeptide, *e.g.*, a transcriptional activator (*e.g.*, a trans-acting factor), a chaperone, and a processing protease. Any factor that is functional in the filamentous fungal cell of choice may be used in the present invention. The nucleic acids encoding one or more of these factors are not necessarily in tandem with the nucleic acid sequence encoding the polypeptide.

An activator is a protein which activates transcription of a nucleic acid sequence encoding a polypeptide ( Kudla et al., 1990, EMBO Journal 9: 1355-1364; Jarai and Buxton, 1994, Current Genetics 26: 2238-244; Verdier, 1990, Yeast 6: 271-297). The nucleic acid sequence encoding an activator may be obtained from the genes encoding *Saccharomyces cerevisiae* heme activator protein 1 (*hap1*), *Saccharomyces cerevisiae* galactose metabolizing protein 4 (*gal4*), *Aspergillus nidulans* ammonia regulation protein (areA), and *Aspergillus oryzae* alpha-amylase activator (*amyR*). For further examples, see Verdier, 1990, *supra* and MacKenzie et al., 1993, Journal of General Microbiology 139: 2295-2307.

A chaperone is a protein which assists another polypeptide in folding properly (Hartl et al., 1994, TIBS 19: 20-25; Bergeron et al., 1994, TIBS 19: 124-128; Demolder et al., 1994, Journal of Biotechnology 32: 179-189; Craig, 1993, Science 260: 1902-1903; Gething and Sambrook, 1992, Nature 355: 33-45; Puig and Gilbert, 1994, Journal of Biological Chemistry 269: 7764-7771; Wang and Tsou, 1993, The FASEB Journal 7: 1515-11157; Robinson et al., 1994, Bio/Technology 1: 381-384; Jacobs et al., 1993, Molecular Microbiology 8: 957-966). The nucleic acid sequence encoding a chaperone may be obtained from the genes encoding *Aspergillus oryzae* protein disulphide isomerase or *Saccharomyces cerevisiae* calnexin, *Saccharomyces cerevisiae* BiP/GRP78, and *Saccharomyces cerevisiae* Hsp70. For further examples, see Gething and Sambrook, 1992, *supra,* and Hartl *et al*., 1994, *supra.*

A processing protease is a protease that cleaves a propeptide to generate a mature biochemically active polypeptide (Enderlin and Ogrydziak, 1994, Yeast 10: 67-79; Fuller et al., 1989, Proceedings of the National Academy of Sciences USA 86: 1434-1438; Julius et al., 1984, Cell 37: 1075-1089; Julius et al., 1983, Cell 32: 839-852; U.S. Patent No. 5,702,934). The nucleic acid sequence encoding a processing protease may be obtained from the genes encoding *Saccharomyces cerevisiae* dipeptidylaminopeptidase, *Saccharomyces cerevisiae* Kex2, *Yarrowia lipolytica* dibasic processing endoprotease *(xpr6),* and *Fusarium oxysporum* metalloprotease (p45 gene).

In a more preferred embodiment, the nucleic acid sequence encoding the transcriptional activator is operably linked to a promoter, or a functional part thereof, which is stronger than the corresponding promoter of the parent cell. In an even more preferred embodiment, the promoter, or a functional part thereof, mediates the expression of a gene encoding an extracellular protease, such as the *Aspergillus oryzae* alkaline protease, *A. oryzae* neutral metalloprotease, *A. niger* aspergillopepsin protease, *Fusarium oxysporum* trypsin-like protease or *F. venenatum* trypsin.

The present invention also relates to a host cell useful for the production of a polypeptide wherein the host cell is a mutant of a parent fungal cell in which the mutant comprises
a) a modification or inactivation of a transcriptional activator of the present invention, or a regulatory sequence thereof, and
b) (i) an inducible promoter operably linked to a nucleic acid sequence encoding a transcriptional activator of the present invention, and (ii) a promoter sequence to which the transcriptional activator can bind, operably linked to a nucleic acid sequence encoding the polypeptide, wherein (i) and (ii) can be introduced simultaneously or sequentially.

The inactive form of the transcriptional activator in (a) above is obtained by inactivation or modification of a nucleic acid sequence present in the cell and necessary for the expression of the native transcriptional activator according to any of the methods as disclosed *supra.* In a preferred embodiment the inactivation or modification is obtained by methods which include, but are not limited to, one or more nucleotide insertions, deletions or substitutions, specific or random mutagenesis, gene replacement or gene interruption, and anti-sense techniques using a nucleotide sequence complementary to the nucleic acid sequence of the transcriptional activator. In another preferred embodiment, the inactive form of the native transcriptional activator is obtained by inactivation or modification of a control sequence required for the expression of the transcriptional activator.

In another preferred embodiment, the nucleic acid sequence encoding the native transcriptional activator has the sequence set forth in SEQ ID NO:1. In another preferred embodiment, the transcriptional activator comprises the polypeptide having the amino acid sequence in SEQ ID NO:3.

The inducible promoter sequence in (b) above may be any promoter sequence, or a functional part thereof, wherein the transcription initiation activity of the promoter can be induced according to the fermentation conditions. Preferably, the induction is mediated by a carbon or nitrogen catabolite. In a preferred embodiment, the promoter is the *amdS* promoter of *Aspergillus nidulans* or *A. oryzae,* the *niaD* promoter of *A. nidulans, A. oryzae* or *A. niger,* the *niiA* promoter of *Aspergillus* species, the alkaline phosphatase promoter of *Aspergillus* sp., the acid phosphatase promoter of *Aspergillus* sp., or the *alcA* promoter of *A. niger.*

In another preferred embodiment, the host cell further comprises a promoter sequence, wherein the promoter sequence can be activated by the transcriptional activator and is operably linked to the nucleic acid sequence encoding the polypeptide.

The promoter sequence activated by the transcriptional activator of the present invention may be any promoter sequence, or a functional part thereof, selected from the group which includes but is not limited to promoters obtained from the genes encoding *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, the NA2-tpi promoter (a hybrid of the promoters from the genes encoding *Aspergillus niger* neutral alpha-amylase and *Aspergillus oryzae* triose phosphate isomerase), and mutant, truncated, and hybrid promoters thereof. Particularly preferred promoters for use in filamentous fungal cells are a promoter, or a functional part thereof, from a protease gene; *e.g.*, from the *Fusarium oxysporum* trypsin-like protease gene (U.S. Patent No. 4,288,627), *Aspegillus oryzae* alkaline protease gene (*alp*)*, Aspergillus niger pacA* gene, *Aspergillus oryzae* alkaline protease gene, *A. oryzae* neutral metalloprotease gene, *A. niger* aspergillopepsin protease gene, or *F. venenatum* trypsin gene.

In another preferred embodiment, the host cell harbours at least one copy of a nucleic acid sequence encoding a polypeptide.

In another preferred embodiment, the host cell which expresses the transcriptional activator of the present invention produces less of one or more native proteases than the parent cell when cultured under identical conditions. The protease(s) may be assayed using any of the methods described above. In a more preferred embodiment, an aliquot from a 48 hour culture media is incubated with ³H-labelled sperm whale myoglobin at pH 4.0 and the radioactivity in the TCA-soluble fraction is measured (van Noort, J.M., *et al., supra).*

The nucleic acid constructs described herein may be introduced into a parent fungal cell according to any of the methods as described *supra* in the section, "Host Cells" to obtain a host cell useful for the production of a polypeptide. In a preferred embodiment the nucleic acid construct is integrated into the chromosome of the cell. In another preferred embodiment the nucleic acid construct is maintained as a self-replicating extra-chromosomal vector.

It will be understood that the methods of the present invention are not limited to a particular order for obtaining the mutant fungal cell. The modification of the second nucleic acid sequence may be introduced into the parent cell at any step in the construction of the cell for the production of a polypeptide.

### Producing a Polypeptide

Another aspect of the present invention relates to methods of producing a polypeptide in a host cell of the present invention, comprising: (a) cultivating the host cell which harbours a gene encoding the polypeptide in a nutrient medium suitable for production of the polypeptide; and (b) recovering the polypeptide from the nutrient medium of the host cell.

In one embodiment, the host cell which is a mutant of a parent fungal cell in which the parent cell comprises one or more nucleic acid sequences encoding a protease, the transcription of which is activated by a transcriptional activator of the present invention, and the mutant cell produces less of the transcriptional activator and the protease(s) than the parent cell when cultured under the same conditions.

In another embodiment, the host cell is a mutant of a parent fungal cell in which the mutant (a) produces more of the transcriptional activator of the present invention as compared to the parent cell when cultured under the same conditions; and (b) comprises a DNA sequence encoding the polypeptide, the transcription of which is activated by the transcriptional activator.

In another embodiment, the host cell is a mutant of a parent fungal cell in which the mutant comprises (a) a modification or inactivation of a transcriptional activator of the present invention or a regulatory sequence thereof, and (b) an inducible promoter operably linked to a nucleic acid sequence encoding a transcriptional activator of the present invention and a promoter sequence to which the transcriptional activator can bind, operably linked to a nucleic acid sequence encoding the polypeptide, wherein (i) and (ii) can be introduced simultaneously or sequentially.

The host cells of the present invention are cultivated in a nutrient medium suitable for production of the polypeptide of interest using methods known in the art. For example, the cells may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art (see, *e.g.*, Bennett, J.W. and LaSure, L., eds., More Gene Manipulations in Fungi, Academic Press, CA, 1991). Suitable media are available from commercial suppliers or may be prepared using published compositions (*e.g.*, in catalogues of the American Type Culture Collection). If the polypeptide is secreted into the nutrient medium, the polypeptide can be recovered directly from the medium. If the polypeptide is not secreted, it is recovered from cell lysates.

The resulting polypeptide may be isolated by methods known in the art. For example, the polypeptide may be isolated from the nutrient medium by conventional procedures including, but not limited to, centrifugation, filtration, extraction, spray-drying, evaporation, or precipitation. The isolated polypeptide may then be further purified by a variety of procedures known in the art including, but not limited to, chromatography (*e.g.*, ion exchange, affinity, hydrophobic, chromatofocusing, and size exclusion), electrophoretic procedures *(e.g.,* preparative isoelectric focusing, differential solubility (*e.g.*, ammonium sulfate precipitation), or extraction (see, *e.g.,* Protein Purification, J.-C. Janson and Lars Ryden, editors, VCH Publishers, New York, 1989).

The polypeptide may be detected using methods known in the art that are specific for the polypeptide. These detection methods may include use of specific antibodies, formation of an enzyme product, disappearance of an enzyme substrate, or SDS-PAGE. For example, an enzyme assay may be used to determine the activity of the polypeptide. Procedures for determining enzyme activity are known in the art for many enzymes.

In the methods of the present invention, the host cell produces at least about 20% more, preferably at least about 50% more, more preferably at least about 100% more, even more preferably at least about 200% more, and most preferably at least about 300% more of the polypeptide than a corresponding parent cell when cultivated under the same conditions.

The polypeptide may be any polypeptide whether native or heterologous to the mutant filamentous fungal cell. The term "heterologous polypeptide" is defined herein as a polypeptide which is not produced by a cell. The term "polypeptide" is not meant herein to refer to a specific length of the encoded produce and therefore encompasses peptides, oligopeptides and proteins. The polypeptide may also be a recombinant polypeptide which is a polypeptide native to a cell, which is encoded by a nucleic acid sequence which comprises one or more control sequences, foreign to the nucleic acid sequence, which are involved in the production of the polypeptide. The polypeptide may be a wild-type polypeptide or a variant thereof. The polypeptide may also be a hybrid polypeptide which contains a combination of partial or complete polypeptide sequences obtained from at least two different polypeptides where one or more of the polypeptides may be heterologous to the cell. Polypeptides further include naturally occurring allelic and engineered variations of the above mentioned polypeptides.

In a preferred embodiment, the polypeptide is an antibody or portions thereof, an antigen, a clotting factor, an enzyme, a hormone or a hormone variant, a receptor or portions thereof, a regulatory protein, a structural protein, a reporter, or a transport protein.

In a more preferred embodiment, the enzyme is an oxidoreductase, transferase, hydrolase, lyase, isomerase, or ligase.

In an even more preferred embodiment, the enzyme is an aminopeptidase, amylase, carbohydrase, carboxypeptidase, catalase, cellulase, chitinase, cutinase, deoxyribonuclease, dextranase, esterase, alpha-galactosidase, beta-galactosidase, glucoamylase, alpha-glucosidase, beta-glucosidase, haloperoxidase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

In another even more preferred embodiment, the polypeptide is human insulin or an analog thereof, human growth hormone, erythropoietin, or insulinotropin.

The nucleic acid sequence encoding a heterologous polypeptide may be obtained from any prokaryotic, eukaryotic, or other source. For purposes of the present invention, the term "obtained from" as used herein in connection with a given source shall mean that the polypeptide is produced by the source or by a cell in which a gene from the source has been inserted.

In the methods of the present invention, the mutant filamentous fungal cells may also be used for the recombinant production of polypeptides which are native to the cell. The native polypeptides may be recombinantly produced by, *e.g.*, placing a gene encoding the polypeptide under the control of a different promoter to enhance expression of the polypeptide, to expedite export of a native polypeptide of interest outside the cell by use of a signal sequence, and to increase the copy number of a gene encoding the polypeptide normally produced by the cell. The present invention also encompasses, within the scope of the term "heterologous polypeptide", such recombinant production of polypeptides native to the cell, to the extent that such expression involves the use of genetic elements not native to the cell, or use of native elements which have been manipulated to function in a manner that do not normally occur in the filamentous fungal cell. The techniques used to isolate or clone a nucleic acid sequence encoding a heterologous polypeptide are known in the art and include isolation from genomic DNA, preparation from cDNA, or a combination thereof. The cloning of the nucleic acid sequences from such genomic DNA can be effected, *e.g.*, by using the well known polymerase chain reaction (PCR). See, for example, Innis et al., 1990, PCR Protocols: A Guide to Methods and Application, Academic Press, New York. The cloning procedures may involve excision and isolation of a desired nucleic acid fragment comprising the nucleic acid sequence encoding the polypeptide, insertion of the fragment into a vector molecule, and incorporation of the recombinant vector into the mutant fungal cell where multiple copies or clones of the nucleic acid sequence will be replicated. The nucleic acid sequence may be of genomic, cDNA, RNA, semisynthetic, synthetic origin, or any combinations thereof.

In the methods of the present invention, heterologous polypeptides may also include fused or hybrid polypeptides in which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide. Techniques for producing fusion polypeptides are known in the art, and include, ligating the coding sequences encoding the polypeptides so that they are in frame and expression of the fused polypeptide is under control of the same promoter(s) and terminator. The hybrid polypeptides may comprise a combination of partial or complete polypeptide sequences obtained from at least two different polypeptides wherein one or more may be heterologous to the mutant fungal cell.An isolated nucleic acid sequence encoding a heterologous polypeptide of interest may be manipulated in a variety of ways to provide for expression of the polypeptide. Expression will be understood to include any step involved in the production of the polypeptide including, but not limited to, transcription, post-transcriptional modification, translation, post-translational modification, and secretion. Manipulation of the nucleic acid sequence encoding a polypeptide prior to its insertion into a vector may be desirable or necessary depending on the expression vector. The techniques for modifying nucleic acid sequences utilizing cloning methods are well known in the art.

The present invention is further described by the following examples which should not be construed as limiting the scope of the invention.

### EXAMPLES

### Materials

Chemicals used as buffers and substrates were commercial products of at least reagent grade.

### Strains

AB4.1: a strain of *Aspergillus niger* which is a *cspA1 pyrG1* derivative of strain ATTC 9029 (van Hartingsveldt, W., et al., 1987. Mol. Gen. Genet. 206:71-75; Bos, C.J., et al., Curr. Genet. 14:437-443)

AB1.13: a protease deficient strain of *Aspergillus niger* derived from UV mutagenesis of AB4.1 (Mattern, I.E., et al., 1992. Mol. Gen. Genet. 234:332-336)

13PAP2: an AB1.13 derivative containing multiple copies of the *A. nidulans amdS* gene (Corrick, R. A., et al, 1987. Gene 53: 63 - 71) under control of the *pepA* promoter of *A. niger* (Jarai G. and Buxton F. 1994. Curr Genet 26:238-244). The strain has a protease deficient phenotype and is unable to grow on medium containing acetamide as the sole nitrogen source. Strain 13PAP2 has been deposited at DSM under the name DSM No. 12298.

4PAP6: an AB4.1 derivative containing multiple copies of the of *A. nidulans amdS* gene under control of the *pepA* promoter of *A. niger.* The strain does not have a protease deficient phenotype and is able to grow on medium containing acetamide as the sole nitrogen source.

N402: a strain of *Aspergillus niger,* deposited at the ATCC (Manassas VA, USA) as ATCC Number: 64974

MC1046: a strain of *E. coli,* deposited at the ATCC as ATCC Number: 35467

### Plasmids

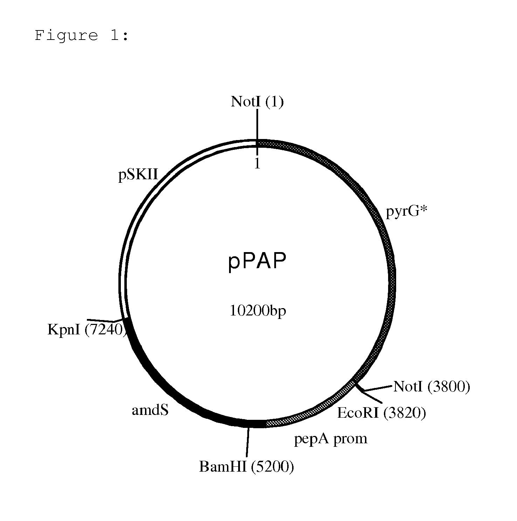

pPAP: constructed as described below in Example 1 and shown in Figure 1

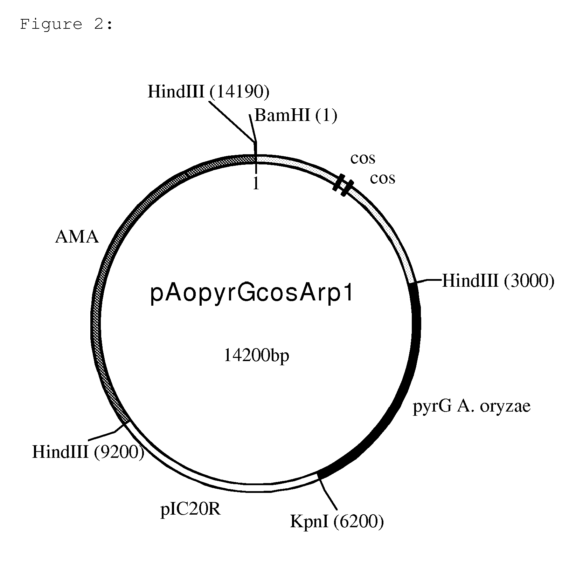

pAopyrGcosArp1: constructed as described below in Example 1 and shown in Figure 2

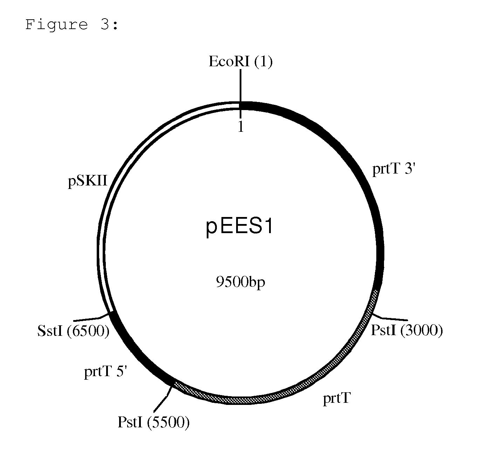

pEES1: constructed as described below in Example 1 and shown in Figure 3

p3SR2: contains the *A. nidulans amdS* gene as described by C.M. Corrick, A.P. Twomey, and M.J. Hynes (1987. Gene 53: 63-71)

pABPYRG*-Not: contains an inactivated *pyrG* gene as described by Verdoes, J.C., et al. (1994. Gene 145: 179-187)

pHelp1: contains the *pyrG* gene from *A. oryzae* as a selective marker and the AMA1 sequences which enable autonomous replication in *A. niger*, cloned into the *E. coli* vector pIC20R, as described by Gems, D., et al. (1991. Gene 98: 61-67)

pAnscos1: contains two cos sites as described by Osiewacz, H.D. (1994. Curr. Genet. 26: 87-90)

pA04-2: contains the *A. oryzae pyrG* gene as described by De Ruiter-Jacobs, Y.M.J.T., et al. (1989. Curr. Genet. 16: 159-163)

pA04-13: contains the *A. oryzae pyrG* gene as described by De Ruiter-Jacobs, Y.M.J.T., et al. (1989. Curr. Genet. 16:159-163)

pUC19: as described by Yanisch-Perron, C., Vieira, J. and Messig, J. (1985, Gene 33:103-119)

### Example 1: Cloning of the prtT transcriptional activator from A. niger

*prtT* was cloned from 13PAP2, an *A. niger* mutant strain which is unable to express the *amdS* gene regulated by the *pepA* protease gene promoter and has a protease deficient phenotype (prt⁻) .

### Construction of the A. niger 13PAP2 reporter strain

The plasmid pPA1 was contructed by ligation of the following three fragments:
1) the *E*. *coli* vector pBlueScript II SK (Stratagene Cloning Systems, La Jolla CA, USA) digested with EcoRI and KpnI;
2) a 1.4 kb EcoRI/BamHI restriction fragment containing the 1.2 kb promoter region of the *pepA* gene linked to about 130 bp of the *amdS* coding sequence from the start codon to an internal BamHI site, amplified by PCR; and
3) a 2.1 kb BamHI/KpnI fragment from p3SR2 which contains most of the *A. nidulans amdS* gene

Fragment 2 was constructed in two steps. In a first step genomic DNA from *A. niger* N402 prepared from protoplasts as described below in the section "Construction of the Cosmid Library" was used as the template, and the two oligonucleotides shown below, pepApr and pepA/amdS, were used as primers:
PepApr: CGG AAT TCG CAT GCT GGA GGT GCT TCT AA
pepA/amdS: TTC CCA GGA TTG AGG CAT TTT GAC CAC GAG AAT

The 1200 bp PCR product obtained from this reaction was then used as a primer in a second PCR reaction together with the oligonucleotide MBL1213 shown below, and plasmid p3SR2 as the template.
MBL1213: TAA CTT CCA CCG AGG TC

The product obtained by ligation of the three fragments described above was subsequently transfected into *E. coli* DH5α.

In the final construction procedure, pPA1 was digested with NotI and ligated to a 3.8 kb NotI fragment from pABPYRG*-Not, resulting in plasmid pPAP which is shown in Fig. 1. pPAP was transformed into *A. niger* AB 1.13, and a transformant with pPAP integrated into the *pyrG* locus in multicopy was isolated. A spontanous 5 flourotic acid (FOA) resistant, uridine-requiring mutant of this transformant that could be complemented with the *pyrG* gene was named 13PAP2.

### Construction of pAopyrGcosArp1

The plasmid pAopyrGcosArp1 was constructed by ligation and subsequent transfection into *E*. *coli* DH5a of the following three fragments:
1) the *E. coli* vector pHelp1 cut with Acc65I and BamHI
2) a 3.0 kb BamHI/HindIII fragment from pAnscos1 containing two cos sites
3) a 3.2 kb Acc65I/HindIII fragment from pA04-2 containing the *A. oryzae pyrG* gene

The resulting plasmid, pAopyrGcosArp1, is self-replicating in *Aspergilli* and can be selected for by growth on medium lacking uridine. pAopyrGcosArp1 is depicted in Fig. 2.

### Construction of the cosmid library

A cosmid library of *Aspergillus niger* was constructed using the "SuperCos1 cosmid vector kit" (Stratagene Cloning Systems, La Jolla CA, USA) according to the supplier's instructions.

Genomic DNA from *A. niger* N402 was prepared from protoplasts made by standard procedures.

After isolation the protoplasts were pelleted by centrifugation at 2000 rpm for 10 minutes in a Beckman GS-6R; the pellet was then suspended in a buffer containing 22.5 mM triisonaphtalene sulphonic acid, 275 mM para-aminosalicylic acid, 0.2 M Tris-HCl (pH 8.5), 0.25 M NaCl and 50 mM EDTA immediately followed by addition of 1 volume of phenol/chloroform (1:1). After careful mixing and centrifugation at 3000 rpm for 20 minutes the aqueous phase was decanted and DNA was precipitated using standard procedures.

The size of the genomic DNA was analysed by electrophoresis on a 0.3% agarose gel run for 20 hours at 30 volts at 4°C. The ethidium bromide stained gel showed that the recovered DNA ranged in size from 50 to greater than 100 kb. The DNA was partially digested using MboI. The size of the digested DNA was 30 to 50 kb as determined by the same type of gel analysis as above. The pAopyrGcosArp1 vector, purified using a kit from QIAGEN (Venlo, The Netherlands) following the manufacturer's instructions, was digested with BamHI, dephosphorylated and gel purified. Ligation and packaging were performed following standard procedures.

After titration of the library, all of the packaging mix from a single ligation and packaging was transfected into the host cell, MC1046, and plated on 50 µg/ml ampicillin LB plates. Approximately 40,000 colonies were obtained. Cosmid preparations from 10 colonies showed that they all had inserts of the expected size. The 40,000 colonies were then soaked in LB medium and scraped off of the plates, then aliquoted for storage in 15% glycerol at -80°C. This represents an approximate 40-fold amplification of the *A. niger* genome.

### Selection of prtT clones

Cosmid DNA was prepared from the library and introduced into 13PAP2 according to the transformation procedure described by P.J. Punt and C.A.M.J.J. Van Den Hondel (1992. Methods Enzymol 216: 447-457). Repeated efforts to select for the *pyrG* marker only resulted in a recovery of between 4000 to 30,000 transformants. A double selection for the *pyrG* marker and growth on medium containing acetamide as the sole nitrogen source resulted in a total of 65 primary transformants from five different experiments.

Each primary transformant was screened for protease activity, growth on medium containing acetamide as the sole nitrogen source and instability of the these two characteristics. An acetamidase⁺ phenotype, screened by growth on medium containing acetamide, is an indication of acetamidase activity resulting from activation of the *pepA* promoter in the reporter cassette in which the *pepA* promoter is linked to the *amdS* coding sequence. A protease⁺ phenotype was screened using minimal medium plates containing dialyzed skim milk as the sole nitrogen source (Mattern, I.E., et al., 1992. Mol Gen Genet 234:332-336). On these plates the wild-type AB4.1 strain makes a clear halo whereas the AB1.13 mutant produces a very small halo. This difference is not due to differences in the activity of *pepA* since a *pepA* deleted strain can also produce a large halo on these plates. Therefore, a large halo on milk plates indicates activation of other extracellular proteases.

Instability was tested by growing diluted spore stocks on medium containing uridine. Single-spore-derived colonies were picked from these plates and tested for protease activity and growth on acetamide. The screening results revealed that in more than 70% of the colonies both characteristics were lost. Therefore, the two phenotypes were either lost or retained together, indicating that activation of the *pepA* promoter and other protease promoters is coordinately regulated and linked to the presence of the *pyrG* marker. The gene responsible for this phenotype was named *prtT.* Twelve acetamidase⁺, protease⁺ transformants were then isolated.

### Isolation of the prtT gene

In order to rescue the *prtT* gene from the acetamidase⁺, protease⁺ transformants of 13PAP2, DNA was prepared from mycelium grown in minimal medium as previously described. This DNA was used in an attempt to transform competent *E*. *coli* DH5a cells. Several hundreds of ampicillin-resistant colonies were obtained. DNA analysis showed they all contained sequences derived from the pHelp1 plasmid. Cosmid DNA isolated from *E. coli* colonies was then retransformed into 13PAP2. Two DNA samples gave rise to transformants which showed both growth on acetamide containing medium and increased protease activity. DNA from one of the cosmids, ACR1, was then digested with several restriction enzymes. The resulting fragments were then co-transformed with pAopyrGcosArp1 into strain 13PAP2. EcoRI, PstI, BamHI and KpnI digestion of ACR1 gave rise to transformants capable of growth on acetamide and high protease activity, whereas SalI and HindIII digests did not. Because EcoRI digestion gave the simplest pattern, separate EcoRI fragments were gel-isolated and with pAopyrGcosArp1 used to co-transform 13PAP2. Only one fragment, a 15 kb EcoRI fragment, gave rise to transformants capable of growth on acetamide-containing medium. This fragment was subcloned in pBluescript II SK in order to subclone *prtT* from the cosmid. Since the insert of this clone was still rather large, separate PstI bands were gel isolated and each was co-transformed with pAopyrGcosArp1 into 13PAP2. Only one band, a 2.5 kb PstI fragment, gave rise to transformants that could grow on acetamide-containing medium. This fragment was subcloned in pBlueScript II SK. Four subclones, ClE 0.7, ClE 1.8, NcE 1.1 and NcE 1.4, were constructed from this plasmid based on the restriction map. In addition, a 6.5 kb SstI/EcoRI fragment encompassing the 2.5 kb PstI fragment was subcloned, resulting in pEES1 (shown in Fig. 3).

Southern blot analysis of genomic DNA from AB4.1 showed the presence of only one copy of *prtT.*

### Example 2: Sequencing of the prtT gene and analysis of the sequence

All sequence reactions were prepared using dRhodamine Terminator Cycle Sequencing Kits or BigDye^{™} Terminator Cycle Sequencing Kits from the Perkin-Elmer Corporation (Branchburg NJ, USA). The reactions were run on an ABI PRISM^{®} 377 DNA Sequencer (Perkin-Elmer Corporation) following the manufacturer's instructions.

The *prtT* gene was sequenced from the genomic clones ClE 0.7, ClE 1.8, NcE 1.1, NcE 1.4 and pEES1. The sequence specific primers used are listed below:

| | | | | | | | | |
|---|---|---|---|---|---|---|---|---|
| 122958: | CGA | TCG | ATG | ACT | GCC | TGT | | |
| 122956: | AGA | GAC | ACA | TAG | TGC | CTT | | |
| 122959: | GCT | TAT | AGT | CGA | TAG | CGC | | |
| 122960: | CCT | CTC | TCC | AGC | GAT | GGT | | |
| 122962: | ATG | GAA | TAC | ATA | CTG | CTT | | |
| 122961: | ATG | AAA | CCC | ACT | GTA | GCT | | |
| 122963: | TGC | TCG | ATA | AGC | GGG | TCC | | |
| 122964: | AAT | CTT | ATG | GAC | CCG | CTT | | |
| 124289: | CCC | CGG | GAA | ACA | AGA | ACA | GG | |
| 124290: | GTT | GGC | GGA | CCT | TGA | CTA | TG | |
| 125112: | ACA | GCT | ACA | GTG | GGT | TTC | ATC | T |
| 125111: | AGT | CAA | CGG | GGG | AAG | TCT | C | |
| 128330: | CTA | GCA | GCG | TAT | CGG | TCA | GC | |
| 130887: | CTT | GGA | AAA | GAA | ACG | ATA | G | |
| 130888: | AAC | GTA | CGC | TTT | CCT | CCT | T | |
| 134135: | GGG | TCC | GTC | CAG | TCC | GTT | CTT | |

| | | | | | | | | |
|---|---|---|---|---|---|---|---|---|
| -48 reverse: | AGC | GGA | TAA | CAA | TTT | CAC | ACA | GGA |
| -40 universal: | GTT | TTC | CCA | GTC | ACG | AC | | |

A mutant allele of the gene was obtained by PCR amplification of genomic DNA isolated from the mutant strain AB1.13 using the following primers:

| | | | | | | | |
|---|---|---|---|---|---|---|---|
| PstI: | TC | ATC | CCT | GGT | GTT | ACT | GC |
| PstII: | C | ATG | GAT | TGG | CTG | GCC | G |

The complete DNA sequence of the *prtT* gene is shown in SEQ ID NO:1. The sequence of the PCR fragment of the mutant allele is shown in SEQ ID NO:4.

Analysing the DNA sequence SEQ ID NO:1 using the computer software Netgene 2 (S.M. Hebsgaard, P.G. Korning, N. Tolstrup, J. Engelbrecht, P. Rouze, S. Brunak (1996. Nucleic Acids Research 24: 3439-3452) suggested the existence of 5 exons (see annotations to SEQ ID NO 1).

### Analysis of prtT cDNA

mRNA was purified from total RNA (isolated according to the DNA isolation method described above in Example 1) using a commercial poly(A)⁺ RNA isolation kit (Pharmacia, Uppsala SE) from a culture of *A. niger* grown under conditions favourable for protease production (J.P.T.W. Van Den Hombergh, et al., 1997. Eur. J. Biochem. 247:605-613). Double stranded cDNA was prepared using standard procedures and used for PCR reactions with the following primers:

| oligo-dT primer: | T₂₀N |
|---|---|
| Prt270n: | TACTCTCCAGATTGCCTG |
| Prt1420r: | TGAGATACCACTCAGCAG |
| prt1350n: | TGCACTTCTCTGTCTCTG |
| Prt2365r: | GACTTCTGGCATCAGTTG |
| prt2320n: | CTCATGGATGGCATGATC |

A PCR reaction with the primers Prt270n and Prt1420r produced a fragment of approximately 1.0 kb. The fragment was cloned into a pGEM-T vector (Promega Corp., Madison WI, USA), and the insert in the resulting plasmid was sequenced using the primers 122958, 122960, -40 universal and -48 reverse. The result confirmed the presence of two introns in this part of the gene.

A second PCR reaction with the primers Prt1350n and Prt2365r produced a fragment of approximately 0.9 kb. This fragment was also cloned in a pGEM-T vector, and the insert in the resulting plasmid was sequenced using the primers 124289, 124290, -40 universal and -48 reverse. The result confirmed the presence of a single intron in this part of the gene.

Another PCR reaction with the oligo-dT primer and primer Prt2320n produced a fragment of approximately 350 bp. This fragment was also cloned in a pGEM-T vector. Sequencing of the insert using primers -40 universal and -48 reverse showed that the fragment contained the 3' part of *prtT* and confirmed the presence of another intron.

The deduced protein sequence of the translated *prtT* gene is shown in SEQ ID NO:2. The deduced protein sequence of the translated mutant allele *prt13* is shown in SEQ ID NO:5. A comparison of SEQ ID NO:2 and SEQ ID NO:5 indicates that the only difference between the two is in position 112 where the leucine residue in the translated *prtT* gene is replaced by proline in the translated *prt13* gene.

Analysis of the deduced PrtT protein sequence reveals the presence of a Zinc(II)2Cys6 binuclear cluster DNA binding motif (SEQ ID NO:2, residues 47-81). This motif defines the GAL4 class of fungal transcriptional activators (Reece, M. J., and Ptashne, M. 1993. Science 261: 909-911). The presence of the motif in the *prtT* gene strongly indicates that prtT is a transcriptional activator.

### EXAMPLE 3: Disruption of the prtT gene in a wild-type A. niger strain

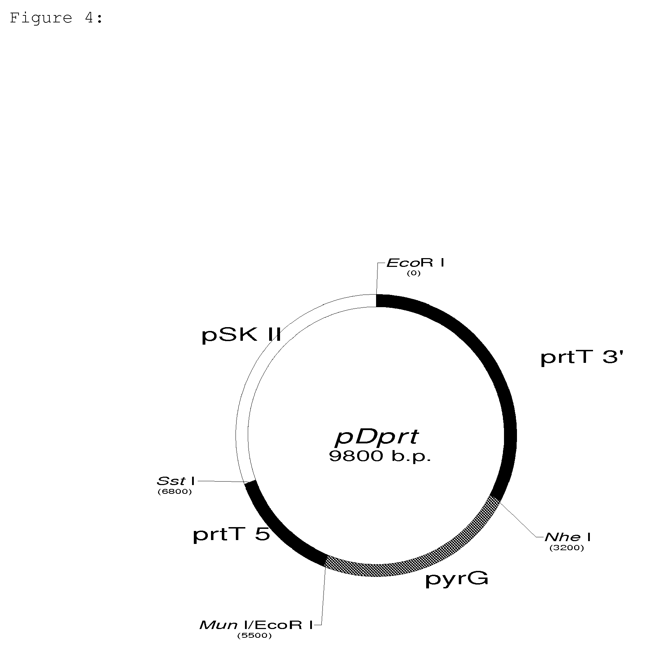

A plasmid was constructed in which the upstream and downstream sequences of the *prtT* gene are separated by the *A. oryzae pyrG* gene. Plasmid pEES1 was digested with MunI and NheI which removed a 2.1 kb fragment containing most of the coding sequence of *prtT.* A 2.3 kb EcoRI/NheI fragment from pA04-13 containing the *A. oryzae pyrG* gene was cloned in the MunI and NheI sites of pEES1. The resulting plasmid, shown in Fig. 4, was named pDprt. This construct was then used to transform *A. niger* strain AB4.1 to uridine prototrophy. About 150 uridine prototrophic transformants were then analyzed for protease activity on skim milk containing plates. Five of these did not make a halo on these plates indicating that protease activity was very low. Comparison of strains with a disrupted *prtT* gene and the mutant AB1.13 strain did not show any differences in protease activity or phenotype.

### EXAMPLE 4: Overexpression of PrtT

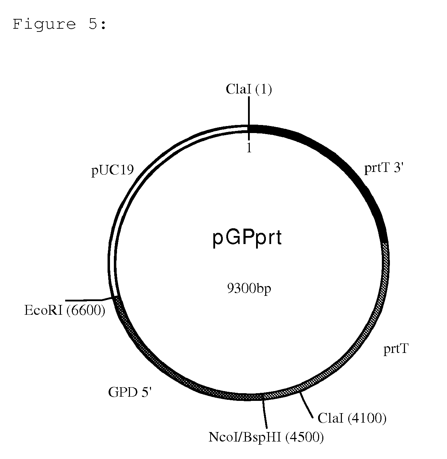

A plasmid, pGPprt, (Figure 5) containing the coding region and 3' noncoding sequences of *prtT* fused to the promoter of the *A. niger gpd* gene was constructed. The *gpd* gene codes for glyceraldehyde-3-phosphate dehydrogenase, a constitutively expressed enzyme involved in primary metabolism. The promoter used was a fragment upstream of the coding region.
The plasmid is transformed into A. niger AB4.1 by cotransformation with the pyrG selection plasmid pA04-13. Transformants with increased prtT transcription as determined by Southern blot analysis is analysed for increased protease expression.

### Deposit of Biological Materials

The following biological material has been deposited under the terms of the Budapest Treaty with the Deutsche Sammlung von Microorganismen und Zellkulturen GmbH, Mascheroder Weg 1b, D-38124 Braunschweig, Germany and given the following accession number:

| Deposit | Accession Number | Date of Deposit |
|---|---|---|
| *Escherichia coli,* pEES | DSM 12294 | 1998-07-14 |
| *Aspergillus niger* 13PAP2 | DSM 12298 | 1998-07-14 |

The strain has been deposited under conditions that assure that access to the culture will be available during the pendency of this patent application. The deposit represents a substantially pure culture of the deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

Various references are cited herein, the disclosures of which are incorporated by reference in their entireties.

## Claims

1. A fungal host cell useful for the production of a polypeptide, wherein the cell:
a) is a mutant of a parent fungal cell in which the parent cell comprises one or more DNA sequences encoding a protease, the transcription of which is activated by a transcriptional activator encoded by a nucleic acid sequence of any of claims 13 or 14; and
b) produces less of the transcriptional activator and the protease(s) than the parent cell when cultured under the same conditions.

2. The host cell of claim 1, wherein the reduced production of the transcriptional activator is obtained by modification or inactivation of a nucleic acid sequence present in the cell and necessary for expression of the transcriptional activator.

3. A fungal host cell useful for the production of a polypeptide, wherein the host cell is a mutant of a parent cell, in which the mutant:
a) produces more of a transcriptional activator encoded by a nucleic acid sequence of any of claims 13 or 14 than the parent cell when cultured under the same conditions, and
b) comprises a DNA sequence encoding the polypeptide, the transcription of which is activated by the transcriptional activator.

4. The host cell of claim 3, wherein the host cell produces more of the transcriptional activator than the parent cell by introducing into the parent cell one or more copies of:
(i) a nucleic acid sequence of any of claims 13 or 14, (ii) the nucleic acid construct of claim 15, or (iii) the expression vector of claim 16, whereby the host cell produces more of the polypeptide than the parent cell when cultured under the same conditions.

5. The host cell of claim 3 or 4, wherein the nucleic acid acid sequence encoding the transcriptional activator is operably linked to a promoter which is stronger than the corresponding promoter of the parent cell.

6. A fungal host cell useful for the production of a polypeptide, wherein the cell is a mutant of a parent cell in which the mutant comprises:
a) a modification or inactivation of a transcriptional activator which is encoded in a native nucleic acid sequence of any of claims 13 or 14, or a regulatory sequence thereof, and
b) (i) an inducible promoter operably linked to a nucleic acid sequence of any of claims 13 or 14, and (ii) a promoter sequence to which a transcriptional activator encoded by the nucleic acid sequence of any of claims 13 or 14 can bind, operably linked to a nucleic acid sequence encoding the polypeptide, wherein (i) and (ii) can be introduced simultaneously or sequentially.

7. The host cell of claim 6, wherein the inducible promoter is selected from the group in which the induction is mediated by a carbon or nitrogen catabolite.

8. The host cell of any of claims 4 to 7 which further comprises a promoter sequence, wherein the promoter sequence can be activated by the transcriptional activator and is operably linked to the nucleic acid sequence encoding the polypeptide.

9. A method of producing a polypeptide, comprising:
(a)cultivating the host cell of any of claims 1 to 8, wherein the host cell harbours a gene encoding the polypeptide, in a nutrient medium suitable for production of the polypeptide; and
(b)recovering the polypeptide from the nutrient medium of the mutant cell.

10. The method of claim 9, wherein the polypeptide is native to the parent cell.

11. The method of claim 9, wherein the polypeptide is heterologous to the parent cell.

12. The method of claim 9, wherein the polypeptide is an antibody or portions thereof, an antigen, a clotting factor, an enzyme, a hormone or a hormone variant, a receptor or portions thereof, a regulatory protein, a structural protein, a reporter, or a transport protein.

13. An isolated nucleic acid sequence encoding a polypeptide having transcriptional activation activity on protease promoters, selected from the group consisting of:
(a) a nucleic acid sequence having at least 70% identity with the nucleic acid sequence of SEQ ID NO:1;
(b) a nucleic acid sequence encoding a polypeptide having an amino acid sequence which has at least 50% identity with the amino acid sequence of SEQ ID NO:2;
(c) a nucleic acid sequence which hybridizes under medium stringency conditions with (i) the nucleic acid sequence of SEQ ID NO:1, or (ii) its complementary strand, wherein the medium stringency conditions are defined by prehybridization and hybridization at 42°C in 5x SSPE, 0.3% SDS, 200 µg/ml sheared and denatured salmon sperm DNA, and 35% formamide, and wash conditions are defined by 50°C for 30 minutes in 2X SSC, 0.2% SDS; and
(d) a subsequence of (a), (b), or (c), wherein the subsequence encodes a polypeptide with the amino acid sequence of SEQ ID NO:3.

14. The nucleic acid sequence of claim 13, wherein the nucleic acid sequence is obtained from a fungal cell or a yeast cell.

15. A nucleic acid construct comprising the nucleic acid sequence of any of claims 13 or 14 operably linked to one or more control sequences which direct the production of the polypeptide in a suitable expression host.

16. An expression vector comprising the nucleic acid construct of claim 15, a promoter, and transcriptional and translational stop signals.

17. A host cell comprising the nucleic acid construct of claim 15 or the expression vector of claim 16.

18. An isolated polypeptide selected from the group consisting of:
(a) a polypeptide which is encoded in a nucleic acid sequence which hybridizes under medium stringency conditions with (i) the nucleic acid sequence of SEQ ID NO:1; (ii) its complementary strand, or (iii) a subsequence of SEQ ID NO:1 which encodes a polypeptide fragment which has transcriptional activation activity, wherein the medium stringency conditions are defined by prehybridization and hybridization at 42°C in 5X SSPE, 0.3% SDS, 200 µg/ml sheared and denatured salmon sperm DNA, and 35% formamide, and wash conditions are defined at 50°C for 30 minutes in 2X SSC, 0.2% SDS;
(b) a polypeptide having an amino acid sequence which has at least 50% identity with the amino acid sequence of SEQ ID NO:2; and
(c) a polypeptide comprising the amino acid sequence of SEQ ID NO:3, or an allelic variant thereof.

19. A method for producing the polypeptide of claim 18 comprising (a) cultivating the host cell of claim 17 under conditions conducive for production of the polypeptide; and (b) recovering the polypeptide.
